# EUROPEAN PATENT SPECIFICATION

(11) **EP 3 370 669 B1**
(45) Date of publication and mention of the grant of the patent: **22.12.2021**
(21) Application number: 16801646.7
(22) Date of filing: 04.11.2016
(51) Int. Cl.: A61F 13/532, A61F 13/534

(54) **THIN AND FLEXIBLE ABSORBENT ARTICLES**
DÜNNE UND FLEXIBLE ABSORBIERENDE ARTIKEL
ARTICLES ABSORBANTS MINCES ET SOUPLES

(30) Priority: 04.11.2015 US 201562251006 P
(43) Date of publication of application: 12.09.2018
(73) Proprietor: The Procter & Gamble Company, Cincinnati, OH 45202 (US)
(72) Inventor: BEWICK-SONNTAG, Christopher Philip, Cincinnati, Ohio 45202 (US); MORROW, Clint Adam, Cincinnati, Ohio 45202 (US); HUBBARD JR., Wade Monroe, Cincinnati, Ohio 45202 (US); DUVAL, Dean Larry, Cincinnati, Ohio 45202 (US); KIRKBRIDE, Tana Marie, Cincinnati, Ohio 45202 (US)
(74) Representative: P&G Patent Germany
(86) International application number: PCT/US2016/060593
(87) International publication number: WO 2017/079606

(56) References cited:
- EP-A1- 2 740 452
- WO-A1-2013/180937
- WO-A1-2014/145326
- US-A1- 2013 079 741

## Description

### FIELD OF THE INVENTION

The present invention relates to absorbent articles which are particularly thin and flexible, and able to retain their shape and move with the body like a garment and nevertheless have an high capacity to absorb fluids and are particularly effective in absorbing these fluids in a quick manner.

This is particularly relevant for products worn on a daily basis and may have to absorb larger amounts of urine discharge and includes products that are worn for both menstrual and incontinence use.

Absorbent articles according to the present invention can be, for example, diapers, incontinent briefs, training pants, diaper holders and liners, sanitary hygiene garments, and the like.

### BACKGROUND OF THE INVENTION

There are two specific challenges in delivering thin, flexible, garment like yet highly absorbent products; the first is having sufficient fluid storing volume within the core system to accommodate larger discharge volumes and the second is maintaining shape and the fluid storage volume under bodily compressive forces while the garment is being worn.

Traditionally, highly absorbent products such as incontinence or heavy menstrual flow products are relatively thick (>6mm) in order to absorb high amounts of discharge delivered quickly.

More recently, thinner products (<6mm) having high absorbency have been developed but these products are invariably stiffer and harder to deform in order to preserve their starting shape and maintain core storage volume under pressure.

With these type of products a further limitation arises, namely during bodily compressive forces the products tend to buckle (plastically deform) and the panty is no longer able to provide sufficient recovery force (via elastics and material stretch during motion) to unbuckle the plastically deformed shape and to return the product to the desired shape to best absorb fluid and sustain volume to store fluid.

In order to overcome these limitations it has been proposed the use of faster absorbent materials that swell when they absorb fluid such as faster super absorbent polymers as used in baby diapers and traditional incontinence products for adults.

These materials are more dense and swell as they absorb, however they are too slow to absorb during a high discharge incidence of menses and/or urine and typically require bulky acquisition volumes as temporary fluid reservoirs so that fluid can more readily enter and be held until absorbed by the swellable storage material. These acquisition volumes inevitably increase the thickness of the absorbent articles.

Another problem that arises with thin and flexible highly absorbent articles is their inability to retain the desired shape for maximizing the fluid absorption rate and sustaining a comfortable, body form-fitting shape as the users goes about their daily routine, in particular when the absorbent article becomes loaded following repeated insults of urine or menses.

Frequently, in the case of stress or early stages of urge incontinence an absorbent article may be worn over more than one loading incidence. It is therefore important to sustain the desired "garment like" wearing experience, shape stability and absorption properties once loaded so that the women can continue her current activities without fear or the product sagging, or noticeable bulges occurring that are typical of thicker products and baby diapers that may render the article more visible and cause embarrassment to the user.

WO2013/180937 A1 discloses (see abstract) an absorbent composite comprises a first absorbent layer comprising a polymeric foam having an average cell size of at least 100 microns and discrete pieces of superabsorbent polymer dispersed within the polymeric foam; and a second absorbent layer in fluid communication with the first absorbent layer. The second absorbent material in the second layer can be made of a variety of materials. In some embodiments, the second absorbent material is the same or similar (e.g. polyurethane) polymeric foam having more SAP than the first absorbent layer. In another embodiment, the second absorbent layer comprises fibrous materials, typically in the form of a fibrous web.

A technical objective of the present invention is therefore to provide absorbent articles which are thin, flexible, garment fitting and which are able to sustain their shape and absorption speed properties while loaded in a sustained way.

The problems has been inventively solved by identifying certain properties of the absorbent articles which are relatively easy to measure and to modify a given structure and identifying ranges of values for these properties which, alone or even more effectively in combination, are the optimal ranges for providing absorbent articles which solve the technical problem explained above to a superior extent if compared with the prior art solutions.

### SUMMARY OF THE INVENTION

The present invention relates to an absorbent article comprising a fluid permeable topsheet, a backsheet and an absorbent element disposed between topsheet and backsheet, wherein the absorbent article has a caliper expansion, measured at five minutes according to the dynamic caliper expansion test described herein, and a dry peak stiffness measured according to the bunch compression test described herein; wherein a ratio of the caliper expansion to the dry peak stiffness is at least 0.5%/N. Further features of the invention are as indicated in claim 1.

### BRIEF DESCRIPTION OF THE DRAWINGS

While the specification concludes with claims particularly pointing out and distinctly claiming the subject matter of the present invention, it is believed that the invention can be more readily understood from the following description taken in connection with the accompanying drawings, in which:
FIG. 1 is a perspective view of one embodiment of a sanitary napkin.
FIG. 2 is a cross-sectional view of the sanitary napkin of Fig. 1, taken through line 2-2.
FIG. 3 is a cross-sectional view of the sanitary napkin of Fig. 1, taken through line 3-3.
FIG. 4 is an SEM micrograph of a heterogeneous mass.
FIG. 5 is an SEM micrograph of a heterogeneous mass.
FIG. 6 is a top view of an alternative pattern.
FIG. 7a-c show top views of alternative patterns.
FIG. 8a-c show top views of alternative patterns.
FIGS. 9A-B are a schematic view of the equipment to perform the Dynamic Caliper Expansion test.
FIG. 10 is a schematic view of the equipment to perform the Dynamic Caliper Expansion test.
FIG. 11 is a schematic view of the equipment to perform the SABAP test.
FIGS. 12A-B is a schematic view of the equipment to perform the SABAP test.
FIG. 13 is a schematic view of the equipment to perform the Bunch Compression test.
FIGS. 14A-B are a schematic view of the equipment to perform the Bunch Compression test.
FIGS. 15A-B are a representative curve from the Bunch Compression test method.

### DETAILED DESCRIPTION OF THE INVENTION

As used herein the term "Absorbent articles" refers to devices that absorb and contain body exudates, such as urine, menses, and feces. The term "disposable" is used herein to describe absorbent articles which are not intended to be laundered or otherwise restored or reused as an absorbent article after a single use. Examples of absorbent articles include diapers, toddler training pants, adult incontinence garments, and feminine hygiene garments such as sanitary napkins, pantiliners, interlabial devices, hemorrhoid pads, body applied pad, and the like. Absorbent articles may be applied to the body or applied to an undergarment.

Absorbent articles and components thereof according to the present invention, including the topsheet, backsheet, absorbent core, and any individual layers of these components, have a body-facing surface and a garment-facing surface. As used herein, "body-facing surface" means that surface of the article or component which is intended to be worn toward or adjacent to the body of the wearer, while the "garment-facing surface" is on the opposite side and is intended to be worn toward or placed adjacent to the wearer's garment when the disposable absorbent article is worn.

In general, the absorbent articles of the present invention comprise a topsheet, a backsheet, and an absorbent "core" or "element" disposed between the topsheet and backsheet and eventually other optional intermediate layers such as, typically, an acquisition/distribution layer positioned between topsheet and core.

As used herein, the term "absorbent core structure" refers to an absorbent core that is has two or more absorbent core layers. Each absorbent core layer is capable of retaining fluid.

As used herein, the term "bicomponent fibers" refers to fibers which have been formed from at least two different polymers extruded from separate extruders but spun together to form one fiber. Bicomponent fibers are also sometimes referred to as conjugate fibers or multicomponent fibers. The polymers are arranged in substantially constantly positioned distinct zones across the cross-section of the bicomponent fibers and extend continuously along the length of the bicomponent fibers. The configuration of such a bicomponent fiber may be, for example, a sheath/core arrangement wherein one polymer is surrounded by another, or may be a side-by-side arrangement, a pie arrangement, or an "islands-in-the-sea" arrangement.

As used herein, the term "biconstituent fibers" refers to fibers which have been formed from at least two polymers extruded from the same extruder as a blend. Biconstituent fibers do not have the various polymer components arranged in relatively constantly positioned distinct zones across the cross-sectional area of the fiber and the various polymers are usually not continuous along the entire length of the fiber, instead usually forming fibrils which start and end at random. Biconstituent fibers are sometimes also referred to as multiconstituent fibers.

As used herein, an "enrobeable element" refers to an element that may be enrobed by the foam. The enrobeable element may be, for example, a fiber, a group of fibers, a tuft, or a section of a film between two apertures. It is understood that other elements are contemplated by the present invention.

A "fiber" as used herein, refers to any material that can be part of a fibrous structure. Fibers can be natural or synthetic. Fibers can be absorbent or non-absorbent.

A "fibrous structure" as used herein, refers to materials which can be broken into one or more fibers. A fibrous structure can be absorbent or adsorbent. A fibrous structure can exhibit capillary action as well as porosity and permeability.

As used herein, the term "meltblowing" refers to a process in which fibers are formed by extruding a molten thermoplastic material through a plurality of fine, usually circular, die capillaries as molten threads or filaments into converging high velocity, usually heated, gas (for example air) streams which attenuate the filaments of molten thermoplastic material to reduce their diameter. Thereafter, the meltblown fibers are carried by the high velocity gas stream and are deposited on a collecting surface, often while still tacky, to form a web of randomly dispersed meltblown fibers.

As used herein, the term "monocomponent" fiber refers to a fiber formed from one or more extruders using only one polymer. This is not meant to exclude fibers formed from one polymer to which small amounts of additives have been added for coloration, antistatic properties, lubrication, hydrophilicity, etc. These additives, for example titanium dioxide for coloration, are generally present in an amount less than about 5 weight percent and more typically about 2 weight percent.

As used herein, the term "non-round fibers" describes fibers having a non-round cross-section, and includes "shaped fibers" and "capillary channel fibers." Such fibers can be solid or hollow, and they can be tri-lobal, delta-shaped, and are preferably fibers having capillary channels on their outer surfaces. The capillary channels can be of various cross-sectional shapes such as "U-shaped", "H-shaped", "C-shaped" and "V-shaped". One practical capillary channel fiber is T-401, designated as 4DG fiber available from Fiber Innovation Technologies, Johnson City, TN. T-401 fiber is a polyethylene terephthalate (PET polyester).

As used herein, the term "nonwoven web" refers to a web having a structure of individual fibers or threads which are interlaid, but not in a repeating pattern as in a woven or knitted fabric, which do not typically have randomly oriented fibers. Nonwoven webs or fabrics have been formed from many processes, such as, for example, meltblowing processes, spunbonding processes, spunlacing processes, hydroentangling, airlaying, and bonded carded web processes, including carded thermal bonding. The basis weight of nonwoven fabrics is usually expressed in grams per square meter (gsm). The basis weight of the laminate web is the combined basis weight of the constituent layers and any other added components. Fiber diameters are usually expressed in microns; fiber size can also be expressed in denier, which is a unit of weight per length of fiber. The basis weight of laminate webs suitable for use in an article of the present invention can range from 10 gsm to 100 gsm, depending on the ultimate use of the web.

As used herein, the term "polymer" generally includes, but is not limited to, homopolymers, copolymers, such as for example, block, graft, random and alternating copolymers, terpolymers, etc., and blends and modifications thereof. In addition, unless otherwise specifically limited, the term "polymer" includes all possible geometric configurations of the material. The configurations include, but are not limited to, isotactic, atactic, syndiotactic, and random symmetries.

As used herein, "spunbond fibers" refers to small diameter fibers which are formed by extruding molten thermoplastic material as filaments from a plurality of fine, usually circular capillaries of a spinneret with the diameter of the extruded filaments then being rapidly reduced. Spunbond fibers are generally not tacky when they are deposited on a collecting surface. Spunbond fibers are generally continuous and have average diameters (from a sample size of at least 10 fibers) larger than 7 microns, and more particularly, between about 10 and 40 microns.

As used herein, a "strata" or "stratum" relates to one or more layers wherein the components within the stratum are intimately combined without the necessity of an adhesive, pressure bonds, heat welds, a combination of pressure and heat bonding, hydro-entangling, needlepunching, ultrasonic bonding, or similar methods of bonding known in the art such that individual components may not be wholly separated from the stratum without affecting the physical structure of the other components. The skilled artisan should understand that while separate bonding is unnecessary between the strata, bonding techniques could be employed to provide additional integrity depending on the intended use.

As used herein, a "tuft" or chad relates to discrete integral extensions of the fibers of a nonwoven web. Each tuft can comprise a plurality of looped, aligned fibers extending outwardly from the surface of the web. In another embodiment each tuft can comprise a plurality of non-looped fibers that extend outwardly from the surface of the web. In another embodiment, each tuft can comprise a plurality of fibers which are integral extensions of the fibers of two or more integrated nonwoven webs.

While particular embodiments of the present invention have been illustrated and described, it would be obvious to those skilled in the art that various other changes and modifications can be made without departing from the scope of the invention

### DETAILED DESCRIPTION OF THE INVENTION

As mentioned above the present invention relates to absorbent articles having a number of easily measurable properties in certain defined optimal ranges. Each range of each property provides absorbent articles according to the invention, although the different ranges and preferred ranges can be combined in any manner to develop embodiments of the present invention.

The properties in question are the following:
a) % caliper expansion, measured at five minutes according to the dynamic caliper expansion test,
b) % caliper expansion, measured at one minute according to the dynamic caliper expansion test,
c) dry peak stiffness measured according to the bunch compression test
d) acquisition rate and rewet as measured according to the SABAP test
e) dry caliper
f) % caliper expansion measured at a time of 5 min or 1 min according to the Dynamic Caliper expansion test

The Dynamic Caliper expansion test is performed as described in the methods section below. This parameter defines the increase in caliper of a portion of the absorbent article when it is exposed to a liquid insult in controlled conditions. Absorbent articles according to the present invention may have a % caliper expansion measured at 5 min of at least 75% or at least 125% or of at least 150% or of at least 275% or from 150% to 600% or from 275% to 600% or from 300% to 500% or from 275% to 600%.

While the caliper expansion measured at 5 min of the Dynamic Caliper expansion test has been found to be representative of the total capacity of expansion of an absorbent article, it has also been found that the value of % caliper expansion measured at 1 min during the performance of the same test has an independent value because it indicates how fast the fluid can be absorbed in a given absorbent article.

Absorbent articles according to the present invention may have a percent (%) caliper expansion measured at 1 minute of at least 150% or from 150% to 600% or from 200% to 600% or from 150% to 250%.

### c) "dry peak stiffness" measured according to the Bunch compression test

The Bunch compression test is performed as described in the methods section below. This value of dry peak stiffness indicates the force required to deform the article when compressed between the legs of a wearer. Typically known absorbent articles having low dry peak stiffness are some thin pantyliners products which are comfortable to wear but have low acquisition rate and low capacity to absorb fluids. Absorbent articles according to the present invention instead have a reduced level of stiffness and at the same time a high acquisition rate and/or a high value of the % caliper expansion. Absorbent articles according to the present invention may have a dry peak stiffness equal or lower than 10N, or equal or lower than 7N, or equal or lower than 6N or from 0.5 to 6N, or from 0.5 to 4N or from 0.5 to 2N.

### d) acquisition rate and rewet as measured according to the SABAP test

The SABAP test is performed as described in the methods section below. The value express the ability of the absorbent article to quickly acquire fluids. Typical absorbent articles having high acquisition rate are thick bulky and stiff articles. Absorbent articles according to the present invention instead combine high acquisition rate with low caliper and low stiffness.

Absorbent articles according to the present invention can have an acquisition rate of at least 0.5ml/s or from 1 to 6 ml/s or from 1.5 to 6 ml/s or from 2 to 6 ml/s.

The SABAP test also measures the rewet performance of absorbent articles. In general absorbent articles according to the present invention will have a rewet value equal or lower than 0.1g.

### e) dry caliper

The dry caliper of the absorbent article is measured according to the Dry caliper method described in the method section below. Prior art absorbent articles having low caliper have low capacity to absorb fluids and low absorption speed. Absorbent articles according to the present invention have instead a relatively low caliper but also a high acquisition rate and a high % caliper expansion. Absorbent articles according to the present invention can have a dry caliper equal or lower than 10mm, or equal or lower than 4.5mm, or from 1 to 10mm or from 1 to 4.5mm or from 1 to 3.5mm.

According to the invention, additional relations between some of the parameters mentioned above have been found to be significant in defining an improved performance of an absorbent article, in particular absorbent articles according to the present invention have a ratio of % caliper expansion, measured at five minutes according to the dynamic caliper expansion test to dry peak stiffness measured according to the bunch compression test of at least 0.5%/N, or at least 0.75%/N, or from 0.5 to 5 %/N, or from 0.75 to 4 %/N, or from 0.75 to 3 %/N.

Absorbent articles according to the present invention may also have a ratio of acquisition rate as measured according to the SABAP test to dry peak stiffness measured according to the bunch compression test of at least 0.5ml/Ns, or at least 0.6ml/Ns, or from 0.6 to 2 ml/Ns, or from 0.6 to 3 ml/Ns.

Absorbent articles according to the present invention may also have a ratio between acquisition rate and dry peak stiffness (according to ii), multiplied by their dry caliper of at least 1.7 ml*mm/Ns, or at least of 2 ml*mm/Ns, or from 2 to 87 ml*mm/Ns or from 2.5 to 67 ml*mm/Ns.

Absorbent articles according to the present invention may also have a ratio of dry caliper to % caliper expansion, measured at 1 minute according to the dynamic caliper expansion test, equal or lower than 3mm/%, or equal or lower than 2.5mm/%, or from 1 to 3 mm/% or from 1.5 to 2.8 mm/%.

Absorbent articles according to the present invention may have one or more of the above mentioned parameters and or relations among parameters in the cited ranges, it is in general preferred that an absorbent article has more than one parameter and/or relation among parameters in the claimed ranges.

In one aspect the present invention relates to an absorbent article comprising a fluid permeable topsheet, a backsheet and an absorbent element disposed between topsheet and backsheet, wherein the absorbent article has a caliper expansion, measured at five minutes according to the dynamic caliper expansion test described herein, of at least 275%, or from 275% to 600% or from 300% to 500%. The absorbent article may also have caliper expansion, measured at one minute according to the dynamic caliper expansion test described herein of at least 150% or from 150% to 250%. The dry caliper of the absorbent article may be 10mm or less, or from 1 to 10mm or from 1 to 4.5mm or from 1 to 3.5mm. The dry peak stiffness measured according to the bunch compression test of the absorbent article may be 10N or less, or from 0.5 to 6N or from 0.5 to 4N or from 0.5 to 2N. The acquisition rate measured according to the SABAP test may be at least 0.5 ml/s or from 1 to 6 ml/s, or from 1.5 to 6 ml/s or from 2 to 6 ml/s. The rewet value measured according to the SABAP test may be 0.1g or less.

In another aspect the present invention relates to an absorbent article comprising a fluid permeable topsheet, a backsheet and an absorbent element disposed between topsheet and backsheet, wherein the absorbent article has a dry caliper equal or lower than 4.5 mm and a caliper expansion, measured at five minutes according to the dynamic caliper expansion test described herein, of at least 75% or of at least 150%. The absorbent article of the invention may have a dry caliper from 2 to 4.5mm and a caliper expansion at 5 minutes of from 150 to 600%. The absorbent article may also have caliper expansion, measured at one minute according to the dynamic caliper expansion test of at least 150% or from 150% to 250%. The dry caliper of the absorbent article may be from 1 to 3.5mm. The dry peak stiffness measured according to the bunch compression test of the absorbent article may be 10N or less, or from 0.5 to 6N or from 0.5 to 4N or from 0.5 to 2N. The acquisition rate measured according to the SABAP test may be at least 0.5 ml/s or from 1 to 6 ml/s, or from 1.5 to 6 ml/s or from 2 to 6 ml/s. The rewet value measured according to the SABAP test may be 0.1g or less.

In another aspect the present invention relates to an absorbent article comprising a fluid permeable topsheet, a backsheet and an absorbent element disposed between topsheet and backsheet, wherein the absorbent article has a caliper expansion, measured at five minutes according to the dynamic caliper expansion test described herein, and a dry peak stiffness measured according to the bunch compression test described herein; wherein a ratio of the caliper expansion to the dry peak stiffness is at least 0.5 %/N or at least 0.75 %/N or from 0.5 to 5 %/N or from 0.75 to 4 %/N or from 0.75 to 3 %/N. The absorbent article may also have a caliper expansion, measured at five minutes according to the dynamic caliper expansion test described herein of at least 275% and a dry peak stiffness, measured according to the bunch compression test described herein, of 10N or less. The absorbent article may also have a caliper expansion, measured at sixty seconds or 1 minute according to the dynamic caliper expansion test described herein of at least 75% and a dry peak stiffness, measured according to the bunch compression test described herein, of 6N or less. The absorbent article may also have a caliper expansion, measured at five minutes according to the dynamic caliper expansion test described herein of at least 75% and a dry peak stiffness, measured according to the bunch compression test described herein, of 6N or less. The rewet value measured according to the SABAP test may be 0.1g or less.

In another aspect the present invention relates to an absorbent article comprising a fluid permeable topsheet, a backsheet and an absorbent element disposed between topsheet and backsheet, wherein the absorbent article has a caliper expansion, measured at one minute according to the dynamic caliper expansion test described herein, of at least 150% or from 150 to 600% or from 200 to 600%. The dry caliper of the absorbent article may be 10mm or less, or from 1 to 10mm or from 1 to 4.5mm or from 1 to 3.5mm. The dry peak stiffness measured according to the bunch compression test of the absorbent article may be 10N or less, or from 0.5 to 6N or from 0.5 to 4N or from 0.5 to 2N. The acquisition rate measured according to the SABAP test may be at least 0.5 ml/s or from 1 to 6 ml/s, or from 1.5 to 6 ml/s or from 2 to 6 ml/s. The rewet value measured according to the SABAP test may be 0.1g or less.

In another aspect the present invention relates to an absorbent article comprising a fluid permeable topsheet, a backsheet and an absorbent element disposed between topsheet and backsheet, wherein the absorbent article has a dry caliper and a caliper expansion, measured at one minute according to the dynamic caliper expansion test described herein wherein a ratio of the dry caliper to the caliper expansion is 3 mm/% or less or 2.5 mm/% or less or from 1 to 3 mm/%, or from 1.5 to 2.8 mm/%. The dry caliper of the absorbent article may be 10mm or less, or from 1 to 10mm or from 1 to 4.5mm or from 1 to 3.5mm. The dry peak stiffness measured according to the bunch compression test of the absorbent article may be 10N or less, or from 0.5 to 6N or from 0.5 to 4N or from 0.5 to 2N. The acquisition rate measured according to the SABAP test may be at least 0.5 ml/s or from 1 to 6 ml/s, or from 1.5 to 6 ml/s or from 2 to 6 ml/s. The rewet value measured according to the SABAP test may be 0.1g or less.

In another aspect the present invention relates to an absorbent article comprising a fluid permeable topsheet, a backsheet and an absorbent element disposed between topsheet and backsheet, wherein the absorbent article has an acquisition rate as measured according to the SABAP test described herein and has a dry peak stiffness, measured according to the bunch compression test described herein, wherein a ratio of the acquisition rate to the dry peak stiffness is at least 0.5 ml/N/s or at least 0.6 ml/Ns or from 0.6 to 3 ml/Ns or from 0.6 to 2 ml/Ns. The absorbent article may also have a ratio of the acquisition rate to the dry peak stiffness multiplied by the dry caliper in mm of at least 1.7 ml*mm/Ns or at least 2 ml*mm/Ns, or from 2 to 87 ml*mm/Ns, or from 2.5 to 67 ml*mm/Ns. The acquisition rate measured according to the SABAP test may be at least 0.5 ml/s or from 1 to 6 ml/s, or from 1.5 to 6 ml/s or from 2 to 6 ml/s. The dry peak stiffness measured according to the bunch compression test of the absorbent article may be 10N or less, or from 0.5 to 6N or from 0.5 to 4N or from 0.5 to 2N. The dry caliper of the absorbent article may be 10mm or less, or from 1 to 10mm or from 1 to 4.5mm or from 1 to 3.5mm. The rewet value measured according to the SABAP test may be 0.1g or less.

Absorbent articles according to the present invention can be for example manufactured incorporating within the absorbent element a core structure as described below.

### Absorbent core structure

An absorbent core structure is disclosed. The absorbent core structure has two or more absorbent core layers. The absorbent core layers may be joined or separate. The absorbent core structure is a two layer system wherein the upper layer is heterogeneous mass layer comprising one or more enrobeable elements and one or more discrete open-cell foam pieces. The upper layer heterogeneous mass layer may be a stratum as defined above. The enrobeable elements are fibers. The lower layer is an absorbent layer that comprises superabsorbent polymer. The absorbent core structure may comprise additional layers above and below the absorbent layer that comprises superabsorbent polymer.

The absorbent core structure may comprise a heterogeneous mass layer as those described in US patent application no. 61/988,565, filed May 5, 2014; US patent application no. 62/115,921, filed February 13, 2015; or US patent application no. 62/018,212. The heterogeneous mass layer has a depth, a width, and a height.

The absorbent core structure may comprise a substrate and superabsorbent polymer layer as those described in US patent no. US8,124,827 filed on December 2, 2008 (Tamburro); US application no. 12/718,244 published on September 9, 2010; US application no. 12/754,935 published on October 14, 2010; or US patent no. 8,674,169 issued on March 18, 2014.

The one or more discrete portions of foam pieces enrobe the elements. The discrete portions of foam pieces are open-celled foam. In an embodiment, the foam is a High Internal Phase Emulsion (HIPE) foam.

In the following description of the invention, the surface of the article, or of each component thereof, which in use faces in the direction of the wearer is called wearer-facing surface. Conversely, the surface facing in use in the direction of the garment is called garment-facing surface. The absorbent article of the present invention, as well as any element thereof, such as, for example the absorbent core, has therefore a wearer-facing surface and a garment-facing surface.

The heterogeneous mass layer contains one or more discrete open-cell foam pieces foams that are integrated into the heterogeneous mass comprising one or more enrobeable elements integrated into the one or more open-cell foams such that the two may be intertwined.

The open-cell foam pieces may comprise between 1% of the heterogeneous mass by volume to 99% of the heterogeneous mass by volume, such as, for example, 5% by volume, 10% by volume, 15% by volume, 20% by volume, 25% by volume, 30% by volume, 35% by volume, 40% by volume, 45% by volume, 50% by volume, 55% by volume, 60% by volume, 65% by volume, 70% by volume, 75% by volume, 80% by volume, 85% by volume, 90% by volume, or 95% by volume.

The heterogeneous mass layer may have void space found between the enrobeable elements, between the enrobeable elements and the enrobed elements, and between enrobed elements. The void space may contain gas. The void space may represent between 1% and 95% of the total volume for a fixed amount of volume of the heterogeneous mass, such as, for example, 5%, 10%, 15%, 20%, 25%, 30%, 35%, 40%, 45%, 50%, 55%, 60%, 65%, 70%, 75%, 80%, 85%, 90% of the total volume for a fixed amount of volume of the heterogeneous mass.

The combination of open-cell foam pieces and void space within the heterogeneous mass may exhibit an absorbency of between 10 g/g to 200 g/g of the heterogeneous mass, such as for example, 40 g/g, 60 g/g, 80 g/g, 100 g/g, 120 g/g, 140 g/g 160 g/g 180 g/g or 190 g/g of the heterogeneous mass. Absorbency may be quantified according to the EDANA Nonwoven Absorption method 10.4-02.

The open-cell foam pieces are discrete foam pieces intertwined within and throughout a heterogeneous mass such that the open-cell foam enrobes one or more of the fibers within the mass. The open-cell foam may be polymerized around the enrobeable elements.

In an embodiment, a discrete open-cell foam piece may enrobe more than one enrobeable element. The enrobeable elements may be enrobed together as a bunch. Alternatively, more than one enrobeable element may be enrobed by the discrete open-cell foam piece without contacting another enrobeable element.

In an embodiment, the open-cell foam pieces may enrobe an enrobeable element such that the enrobeable element is enrobed along the enrobeable elements axis for between 5% and 95% of the length along the enrobeable element's axis. For example, a single fiber may be enrobed along the length of the fiber for a distance greater than 50% of the entire length of the fiber. In an embodiment, an enrobeable element may have between 5% and 100% of its surface area enrobed by one or more open-cell foam pieces.

In an embodiment, two or more open-cell foam pieces may enrobe the same enrobeable element such that the enrobeable element is enrobed along the enrobeable elements axis for between 5% and 100% of the length along the enrobeable element's axis.

The open-cell foam pieces enrobe the enrobeable elements such that a layer surrounds the enrobeable element at a given cross section. The layer surrounding the enrobeable element at a given cross section may be between 0.01 mm to 100 mm such as, for example, 0.1 mm, 0.2 mm, 0.3 mm, 0.4 mm, 0.5 mm, 0.6 mm, 0.7 mm, 0.8 mm, 0.9 mm, 1.0 mm, 1.2 mm, 1.4 mm, 1.6 mm, 1.8 mm, 2.0 mm, 2.2 mm, 2.4 mm, 2.6 mm, 2.8 mm, or 3 mm. The layer may not be equivalent in dimension at all points along the cross section of the enrobeable element. For example, in an embodiment, an enrobeable element may be enrobed by 0.5 mm at one point along the cross section and by 1.0 mm at a different point along the same cross section.

The open-cell foam pieces are considered discrete in that they are not continuous throughout the entire heterogeneous mass layer. Not continuous throughout the entire heterogeneous mass layer represents that at any given point in the heterogeneous mass layer, the open-cell absorbent foam is not continuous in at least one of the cross sections of a longitudinal, a vertical, and a lateral plane of the heterogeneous mass layer. In a non-limiting embodiment, the absorbent foam is not continuous in the lateral and the vertical planes of the cross section for a given point in the heterogeneous mass layer. In a non-limiting embodiment, the absorbent foam is not continuous in the longitudinal and the vertical planes of the cross section for a given point in the heterogeneous mass layer. In a non-limiting embodiment, the absorbent foam is not continuous in the longitudinal and the lateral planes of the cross section for a given point in the heterogeneous mass layer.

In an embodiment wherein the open-cell foam is not continuous in at least one of the cross sections of the longitudinal, the vertical, and the lateral plane of the heterogeneous mass, one or both of either the enrobeable elements or the open-cell foam pieces may be bi-continuous throughout the heterogeneous mass.

The open-cell foam pieces may be located at any point in the heterogeneous mass. In a non-limiting embodiment, a foam piece may be surrounded by the elements that make up the enrobeable elements. In a non-limiting embodiment a foam piece may be located on the outer perimeter of the heterogeneous mass such that only a portion of the foam piece is entangled with the elements of the heterogeneous mass.

In a non-limiting embodiment, the open-cell foam pieces may expand upon being contacted by a fluid to form a channel of discrete open-cell foam pieces. The open-cell foam pieces may or may not be in contact prior to being expanded by a fluid.

An open-celled foam may be integrated onto the enrobeable elements prior to being polymerized. The open cell foam pieces may be impregnated prior to polymerization into or onto two or more different enrobeable elements that are combined to create a heterogeneous mixture of enrobeable elements. The two or more different enrobeable elements may be intertwined such that one enrobeable element may be surrounded by multiples of the second enrobeable element, such as, for example by using more than one type of fiber in a mixture of fibers or by coating one or more fibers with surfactant. The two or more different enrobeable elements may be layered within the heterogeneous mass along any of the vertical, longitudinal, and/or lateral planes such that the enrobeable elements are profiled within the heterogeneous mass for an enrobeable element inherent property or physical property, such as, for example, hydrophobicity, fiber diameter, fiber or composition. It is understood that any inherent property or physical property of the enrobeable elements listed is contemplated herein.

In a non-limiting embodiment the open-cell foam pieces may be partially polymerized prior to being impregnated into or onto the enrobeable elements such that they become intertwined. After being impregnated into or onto the enrobeable elements, the open-celled foam in either a liquid or solid state are polymerized to form one or more open-cell foam pieces. The open-celled foam may be polymerized using any known method including, for example, heat, UV, and infrared. Following the polymerization of a water in oil open-cell foam emulsion, the resulting open-cell foam is saturated with aqueous phase that needs to be removed to obtain a substantially dry open-cell foam. Removal of the saturated aqueous phase or dewatering may occur using nip rollers, and vacuum. Utilizing a nip roller may also reduce the thickness of the heterogeneous mass such that the heterogeneous mass will remain thin until the open-cell foam pieces entwined in the heterogeneous mass are exposed to fluid.

Dependent upon the desired foam density, polymer composition, specific surface area, or pore size (also referred to as cell size), the open-celled foam may be made with different chemical composition, physical properties, or both. For instance, dependent upon the chemical composition, an open-celled foam may have a density of 0.0010 g/cc to about 0.25 g/cc. Preferred 0.04 g/cc.

Open-cell foam pore sizes may range in average diameter of from 1 to 800 µm, such as, for example, between 50 and 700 µm, between 100 and 600 µm, between 200 and 500 µm, between 300 and 400 µm.

In some embodiments, the foam pieces have a relatively uniform cell size. For example, the average cell size on one major surface may be about the same or vary by no greater than 10% as compared to the opposing major surface. In other embodiments, the average cell size of one major surface of the foam may differ from the opposing surface. For example, in the foaming of a thermosetting material it is not uncommon for a portion of the cells at the bottom of the cell structure to collapse resulting in a lower average cell size on one surface.

The foams produced from the present invention are relatively open-celled. This refers to the individual cells or pores of the foam being in substantially unobstructed communication with adjoining cells. The cells in such substantially open-celled foam structures have intercellular openings or windows that are large enough to permit ready fluid transfer from one cell to another within the foam structure. For purpose of the present invention, a foam is considered "open-celled" if at least about 80% of the cells in the foam that are at least 1µm in average diameter size are in fluid communication with at least one adjoining cell.

In addition to being open-celled, in certain embodiments foams are sufficiently hydrophilic to permit the foam to absorb aqueous fluids, for example the internal surfaces of a foam may be rendered hydrophilic by residual hydrophilizing surfactants or salts left in the foam following polymerization, by selected post-polymerization foam treatment procedures (as described hereafter), or combinations of both.

In certain embodiments, for example when used in certain absorbent articles, an open-cell foam may be flexible and exhibit an appropriate glass transition temperature (Tg). The Tg represents the midpoint of the transition between the glassy and rubbery states of the polymer.

In certain embodiments, the Tg of this region will be less than about 200° C for foams used at about ambient temperature conditions, in certain other embodiments less than about 90° C. The Tg may be less than 50° C.

The open-cell foam pieces may be distributed in any suitable manner throughout the heterogeneous mass. In an embodiment, the open-cell foam pieces may be profiled along the vertical axis such that smaller pieces are located above larger pieces. Alternatively, the pieces may be profiled such that smaller pieces are below larger pieces. In another embodiment, the open-cell pieces may be profiled along a vertical axis such that they alternate in size along the axis.

In an embodiment the open-cell foam pieces may be profiled along any one of the longitudinal, lateral, or vertical axis based on one or more characteristics of the open-cell foam pieces. Characteristics by which the open-cell foam pieces may be profiled within the heterogeneous mass may include, for example, absorbency, density, cell size, and combinations thereof.

In an embodiment, the open-cell foam pieces may be profiled along any one of the longitudinal, lateral, or vertical axis based on the composition of the open-cell foam. The open-cell foam pieces may have one composition exhibiting desirable characteristics in the front of the heterogeneous mass and a different composition in the back of the heterogeneous mass designed to exhibit different characteristics. The profiling of the open-cell foam pieces may be either symmetric or asymmetric about any of the prior mentioned axes or orientations.

The open-cell foam pieces may be distributed along the longitudinal and lateral axis of the heterogeneous mass in any suitable form. In an embodiment, the open-cell foam pieces may be distributed in a manner that forms a design or shape when viewed from a top planar view. The open-cell foam pieces may be distributed in a manner that forms stripes, ellipticals, squares, or any other known shape or pattern.

In an embodiment, different types of foams may be used in one heterogeneous mass. For example, some of the foam pieces may be polymerized HIPE while other pieces may be made from open-cell foam, such as, for example, polyurethane. The pieces may be located at specific locations within the mass based on their properties to optimize the performance of the heterogeneous mass.

In an embodiment, the open-celled foam is a thermoset polymeric foam made from the polymerization of a High Internal Phase Emulsion (HIPE), also referred to as a polyHIPE. To form a HIPE, an aqueous phase and an oil phase are combined in a ratio between about 8:1 and 140:1. In certain embodiments, the aqueous phase to oil phase ratio is between about 10:1 and about 75:1, and in certain other embodiments the aqueous phase to oil phase ratio is between about 13:1 and about 65:1. This is termed the "water-to-oil" or W:O ratio and can be used to determine the density of the resulting polyHIPE foam. As discussed, the oil phase may contain one or more of monomers, co-monomers, photo-initiators, cross-linkers, and emulsifiers, as well as optional components. The water phase will contain water and in certain embodiments one or more components such as electrolyte, initiator, or optional components.

The open-cell foam can be formed from the combined aqueous and oil phases by subjecting these combined phases to shear agitation in a mixing chamber or mixing zone. The combined aqueous and oil phases are subjected to shear agitation to produce a stable HIPE having aqueous droplets of the desired size. An initiator may be present in the aqueous phase, or an initiator may be introduced during the foam making process, and in certain embodiments, after the HIPE has been formed. The emulsion making process produces a HIPE where the aqueous phase droplets are dispersed to such an extent that the resulting HIPE foam will have the desired structural characteristics. Emulsification of the aqueous and oil phase combination in the mixing zone may involve the use of a mixing or agitation device such as an impeller, by passing the combined aqueous and oil phases through a series of static mixers at a rate necessary to impart the requisite shear, or combinations of both. Once formed, the HIPE can then be withdrawn or pumped from the mixing zone. One method for forming HIPEs using a continuous process is described in U.S. Pat. No. 5,149,720 (DesMarais et al), issued Sep. 22, 1992; U.S. Pat. No. 5,827,909 (DesMarais) issued Oct. 27, 1998; and U.S. Pat. No. 6,369,121 (Catalfamo et al.) issued Apr. 9, 2002.

The emulsion can be withdrawn or pumped from the mixing zone and impregnated into or onto a mass prior to being fully polymerized. Once fully polymerized, the foam pieces and the elements are intertwined such that discrete foam pieces are bisected by the elements comprising the mass and such that parts of discrete foam pieces enrobe portions of one or more of the elements comprising the heterogeneous mass.

Following polymerization, the resulting foam pieces are saturated with aqueous phase that needs to be removed to obtain substantially dry foam pieces. In certain embodiments, foam pieces can be squeezed free of most of the aqueous phase by using compression, for example by running the heterogeneous mass comprising the foam pieces through one or more pairs of nip rollers. The nip rollers can be positioned such that they squeeze the aqueous phase out of the foam pieces. The nip rollers can be porous and have a vacuum applied from the inside such that they assist in drawing aqueous phase out of the foam pieces. In certain embodiments, nip rollers can be positioned in pairs, such that a first nip roller is located above a liquid permeable belt, such as a belt having pores or composed of a mesh-like material and a second opposing nip roller facing the first nip roller and located below the liquid permeable belt. One of the pair, for example the first nip roller can be pressurized while the other, for example the second nip roller, can be evacuated, so as to both blow and draw the aqueous phase out the of the foam. The nip rollers may also be heated to assist in removing the aqueous phase. In certain embodiments, nip rollers are only applied to non-rigid foams, that is, foams whose walls would not be destroyed by compressing the foam pieces.

In certain embodiments, in place of or in combination with nip rollers, the aqueous phase may be removed by sending the foam pieces through a drying zone where it is heated, exposed to a vacuum, or a combination of heat and vacuum exposure. Heat can be applied, for example, by running the foam though a forced air oven, IR oven, microwave oven or radiowave oven. The extent to which a foam is dried depends on the application. In certain embodiments, greater than 50% of the aqueous phase is removed. In certain other embodiments greater than 90%, and in still other embodiments greater than 95% of the aqueous phase is removed during the drying process.

In an embodiment, open-cell foam is produced from the polymerization of the monomers having a continuous oil phase of a High Internal Phase Emulsion (HIPE). The HIPE may have two phases. One phase is a continuous oil phase having monomers that are polymerized to form a HIPE foam and an emulsifier to help stabilize the HIPE. The oil phase may also include one or more photo-initiators. The monomer component may be present in an amount of from about 80% to about 99%, and in certain embodiments from about 85% to about 95% by weight of the oil phase. The emulsifier component, which is soluble in the oil phase and suitable for forming a stable water-in-oil emulsion may be present in the oil phase in an amount of from about 1% to about 20% by weight of the oil phase. The emulsion may be formed at an emulsification temperature of from about 10° C to about 130° C and in certain embodiments from about 50° C to about 100° C.

In general, the monomers will include from about 20% to about 97% by weight of the oil phase at least one substantially water-insoluble monofunctional alkyl acrylate or alkyl methacrylate. For example, monomers of this type may include C₄-C₁₈ alkyl acrylates and C₂-C₁₈ methacrylates, such as ethylhexyl acrylate, butyl acrylate, hexyl acrylate, octyl acrylate, nonyl acrylate, decyl acrylate, isodecyl acrylate, tetradecyl acrylate, benzyl acrylate, nonyl phenyl acrylate, hexyl methacrylate, 2-ethylhexyl methacrylate, octyl methacrylate, nonyl methacrylate, decyl methacrylate, isodecyl methacrylate, dodecyl methacrylate, tetradecyl methacrylate, and octadecyl methacrylate.

The oil phase may also have from about 2% to about 40%, and in certain embodiments from about 10% to about 30%, by weight of the oil phase, a substantially water-insoluble, polyfunctional crosslinking alkyl acrylate or methacrylate. This crosslinking co-monomer, or cross-linker, is added to confer strength and resilience to the resulting HIPE foam. Examples of crosslinking monomers of this type may have monomers containing two or more activated acrylate, methacrylate groups, or combinations thereof. Nonlimiting examples of this group include 1,6-hexanedioldiacrylate, 1,4-butanedioldimethacrylate, trimethylolpropane triacrylate, trimethylolpropane trimethacrylate, 1,12-dodecyldimethacrylate, 1,14-tetradecanedioldimethacrylate, ethylene glycol dimethacrylate, neopentyl glycol diacrylate (2,2-dimethylpropanediol diacrylate), hexanediol acrylate methacrylate, glucose pentaacrylate, sorbitan pentaacrylate, and the like. Other examples of cross-linkers contain a mixture of acrylate and methacrylate moieties, such as ethylene glycol acrylate-methacrylate and neopentyl glycol acrylate-methacrylate. The ratio of methacrylate:acrylate group in the mixed cross-linker may be varied from 50:50 to any other ratio as needed.

Any third substantially water-insoluble co-monomer may be added to the oil phase in weight percentages of from about 0% to about 15% by weight of the oil phase, in certain embodiments from about 2% to about 8%, to modify properties of the HIPE foams. In certain embodiments, "toughening" monomers may be desired which impart toughness to the resulting HIPE foam. These include monomers such as styrene, vinyl chloride, vinylidene chloride, isoprene, and chloroprene. Without being bound by theory, it is believed that such monomers aid in stabilizing the HIPE during polymerization (also known as "curing") to provide a more homogeneous and better formed HIPE foam which results in better toughness, tensile strength, abrasion resistance, and the like. Monomers may also be added to confer flame retardancy as disclosed in U.S. Pat. No. 6,160,028 (Dyer) issued Dec. 12, 2000. Monomers may be added to confer color, for example vinyl ferrocene, fluorescent properties, radiation resistance, opacity to radiation, for example lead tetraacrylate, to disperse charge, to reflect incident infrared light, to absorb radio waves, to form a wettable surface on the HIPE foam struts, or for any other desired property in a HIPE foam. In some cases, these additional monomers may slow the overall process of conversion of HIPE to HIPE foam, the tradeoff being necessary if the desired property is to be conferred. Thus, such monomers can be used to slow down the polymerization rate of a HIPE. Examples of monomers of this type can have styrene and vinyl chloride.

The oil phase may further contain an emulsifier used for stabilizing the HIPE. Emulsifiers used in a HIPE can include: (a) sorbitan monoesters of branched C₁₆-C₂₄ fatty acids; linear unsaturated C₁₆-C₂₂ fatty acids; and linear saturated C₁₂-C₁₄ fatty acids, such as sorbitan monooleate, sorbitan monomyristate, and sorbitan monoesters, sorbitan monolaurate diglycerol monooleate (DGMO), polyglycerol monoisostearate (PGMIS), and polyglycerol monomyristate (PGMM); (b) polyglycerol monoesters of -branched C₁₆-C₂₄ fatty acids, linear unsaturated C₁₆-C₂₂ fatty acids, or linear saturated C₁₂-C₁₄ fatty acids, such as diglycerol monooleate (for example diglycerol monoesters of C18:1 fatty acids), diglycerol monomyristate, diglycerol monoisostearate, and diglycerol monoesters; (c) diglycerol monoaliphatic ethers of -branched C₁₆-C₂₄ alcohols, linear unsaturated C₁₆-C₂₂ alcohols, and linear saturated C₁₂-C₁₄ alcohols, and mixtures of these emulsifiers. See U.S. Pat. No. 5,287,207 (Dyer et al.), issued Feb. 7, 1995 and U.S. Pat. No. 5,500,451 (Goldman et al.) issued Mar. 19, 1996. Another emulsifier that may be used is polyglycerol succinate (PGS), which is formed from an alkyl succinate, glycerol, and triglycerol.

Such emulsifiers, and combinations thereof, may be added to the oil phase so that they can have between about 1% and about 20%, in certain embodiments from about 2% to about 15%, and in certain other embodiments from about 3% to about 12% by weight of the oil phase. In certain embodiments, co-emulsifiers may also be used to provide additional control of cell size, cell size distribution, and emulsion stability, particularly at higher temperatures, for example greater than about 65° C. Examples of co-emulsifiers include phosphatidyl cholines and phosphatidyl choline-containing compositions, aliphatic betaines, long chain C₁₂-C₂₂ dialiphatic quaternary ammonium salts, short chain C₁-C₄ dialiphatic quaternary ammonium salts, long chain C₁₂-C₂₂ dialkoyl(alkenoyl)-2-hydroxyethyl, short chain C₁-C₄ dialiphatic quaternary ammonium salts, long chain C₁₂-C₂₂ dialiphatic imidazolinium quaternary ammonium salts, short chain C₁-C₄ dialiphatic imidazolinium quaternary ammonium salts, long chain C₁₂-C₂₂ monoaliphatic benzyl quaternary ammonium salts, long chain C₁₂-C₂₂ dialkoyl(alkenoyl)-2-aminoethyl, short chain C₁-C₄ monoaliphatic benzyl quaternary ammonium salts, short chain C₁-C₄ monohydroxyaliphatic quaternary ammonium salts. In certain embodiments, ditallow dimethyl ammonium methyl sulfate (DTDMAMS) may be used as a co-emulsifier.

The oil phase may comprise a photo-initiator at between about 0.05% and about 10%, and in certain embodiments between about 0.2% and about 10% by weight of the oil phase. Lower amounts of photo-initiator allow light to better penetrate the HIPE foam, which can provide for polymerization deeper into the HIPE foam. However, if polymerization is done in an oxygen-containing environment, there should be enough photo-initiator to initiate the polymerization and overcome oxygen inhibition. Photo-initiators can respond rapidly and efficiently to a light source with the production of radicals, cations, and other species that are capable of initiating a polymerization reaction. The photo-initiators used in the present invention may absorb UV light at wavelengths of about 200 nanometers (nm) to about 800 nm, in certain embodiments about 200 nm to about 350 nm. If the photo-initiator is in the oil phase, suitable types of oil-soluble photo-initiators include benzyl ketals, α-hydroxyalkyl phenones, α-amino alkyl phenones, and acylphospine oxides. Examples of photo-initiators include 2,4,6-[trimethylbenzoyldiphosphine] oxide in combination with 2-hydroxy-2-methyl-1-phenylpropan-1-one (50:50 blend of the two is sold by Ciba Speciality Chemicals, Ludwigshafen, Germany as DAROCUR^{®} 4265); benzyl dimethyl ketal (sold by Ciba Geigy as IRGACURE 651); α-,α-dimethoxy-α-hydroxy acetophenone (sold by Ciba Speciality Chemicals as DAROCUR^{®} 1173); 2-methyl-1-[4-(methyl thio) phenyl]-2-morpholino-propan-1-one (sold by Ciba Speciality Chemicals as IRGACURE^{®} 907); 1-hydroxycyclohexyl-phenyl ketone (sold by Ciba Speciality Chemicals as IRGACURE^{®} 184); bis(2,4,6-trimethylbenzoyl)-phenylphosphineoxide (sold by Ciba Speciality Chemicals as IRGACURE 819); diethoxyacetophenone, and 4-(2-hydroxyethoxy)phenyl-(2-hydroxy-2-methylpropyl) ketone (sold by Ciba Speciality Chemicals as IRGACURE^{®} 2959); and Oligo [2-hydroxy-2-methyl-1-[4-(1-methylvinyl) phenyl]propanone] (sold by Lamberti spa, Gallarate, Italy as ESACURE^{®} KIP EM.

The dispersed aqueous phase of a HIPE can have water, and may also have one or more components, such as initiator, photo-initiator, or electrolyte, wherein in certain embodiments, the one or more components are at least partially water soluble.

One component of the aqueous phase may be a water-soluble electrolyte. The water phase may contain from about 0.2% to about 40%, in certain embodiments from about 2% to about 20%, by weight of the aqueous phase of a water-soluble electrolyte. The electrolyte minimizes the tendency of monomers, co-monomers, and cross-linkers that are primarily oil soluble to also dissolve in the aqueous phase. Examples of electrolytes include chlorides or sulfates of alkaline earth metals such as calcium or magnesium and chlorides or sulfates of alkali earth metals such as sodium. Such electrolyte can include a buffering agent for the control of pH during the polymerization, including such inorganic counter-ions as phosphate, borate, and carbonate, and mixtures thereof. Water soluble monomers may also be used in the aqueous phase, examples being acrylic acid and vinyl acetate.

Another component that may be present in the aqueous phase is a water-soluble free-radical initiator. The initiator can be present at up to about 20 mole percent based on the total moles of polymerizable monomers present in the oil phase. In certain embodiments, the initiator is present in an amount of from about 0.001 to about 10 mole percent based on the total moles of polymerizable monomers in the oil phase. Suitable initiators include ammonium persulfate, sodium persulfate, potassium persulfate, 2,2'-azobis(N,N'-dimethyleneisobutyramidine)dihydrochloride, and other suitable azo initiators. In certain embodiments, to reduce the potential for premature polymerization which may clog the emulsification system, addition of the initiator to the monomer phase may be just after or near the end of emulsification.

Photo-initiators present in the aqueous phase may be at least partially water soluble and can have between about 0.05% and about 10%, and in certain embodiments between about 0.2% and about 10% by weight of the aqueous phase. Lower amounts of photo-initiator allow light to better penetrate the HIPE foam, which can provide for polymerization deeper into the HIPE foam. However, if polymerization is done in an oxygen-containing environment, there should be enough photo-initiator to initiate the polymerization and overcome oxygen inhibition. Photo-initiators can respond rapidly and efficiently to a light source with the production of radicals, cations, and other species that are capable of initiating a polymerization reaction. The photo-initiators used in the present invention may absorb UV light at wavelengths of from about 200 nanometers (nm) to about 800 nm, in certain embodiments from about 200 nm to about 350 nm, and in certain embodiments from about 350 nm to about 450 nm. If the photo-initiator is in the aqueous phase, suitable types of water-soluble photo-initiators include benzophenones, benzils, and thioxanthones. Examples of photo-initiators include 2,2'-Azobis[2-(2-imidazolin-2-yl)propane]dihydrochloride; 2,2'-Azobis[2-(2-imidazolin-2-yl)propane]disulfate dehydrate; 2,2'-Azobis(1-imino-1-pyrrolidino-2-ethylpropane)dihydrochloride; 2,2'-Azobis[2-methyl-N-(2-hydroxyethyl)propionamide]; 2,2'-Azobis(2-methylpropionamidine)dihydrochloride; 2,2'-dicarboxymethoxydibenzalacetone, 4,4'-dicarboxymethoxydibenzalacetone, 4,4'-dicarboxymethoxydibenzalcyclohexanone,4-dimethylamino-4'-carboxymethoxydibenzalacetone; and 4,4'-disulphoxymethoxydibenzalacetone. Other suitable photo-initiators that can be used in the present invention are listed in U.S. Pat. No. 4,824,765 (Sperry et al.) issued Apr. 25, 1989.

In addition to the previously described components other components may be included in either the aqueous or oil phase of a HIPE. Examples include antioxidants, for example hindered phenolics, hindered amine light stabilizers; plasticizers, for example dioctyl phthalate, dinonyl sebacate; flame retardants, for example halogenated hydrocarbons, phosphates, borates, inorganic salts such as antimony trioxide or ammonium phosphate or magnesium hydroxide; dyes and pigments; fluorescers; filler pieces, for example starch, titanium dioxide, carbon black, or calcium carbonate; fibers; chain transfer agents; odor absorbers, for example activated carbon particulates; dissolved polymers; dissolved oligomers; and the like.

The heterogeneous mass comprises enrobeable elements and discrete pieces of foam. The enrobeable elements are fibers. The enrobeable elements may be a web such as, for example, nonwoven, a fibrous structure, an air-laid web, a wet laid web, a high loft nonwoven, a needlepunched web, a hydroentangled web, a fiber tow, a woven web, a knitted web, a flocked web, a spunbond web, a layered spunbond/ melt blown web, a carded fiber web, a coform web of cellulose fiber and melt blown fibers, a coform web of staple fibers and melt blown fibers, and layered webs that are layered combinations thereof.

The enrobeable elements may be, for example, conventional absorbent materials such as creped cellulose wadding, fluffed cellulose fibers, wood pulp fibers also known as airfelt, and textile fibers. The enrobeable elements may also be fibers such as, for example, synthetic fibers, thermoplastic particulates or fibers, tricomponent fibers, and bicomponent fibers such as, for example, sheath/core fibers having the following polymer combinations: polyethylene/polypropylene, polyethylvinyl acetate/polypropylene, polyethylene/polyester, polypropylene/polyester, copolyester/polyester, and the like. The enrobeable elements may be any combination of the materials listed above and/or a plurality of the materials listed above, alone or in combination.

The enrobeable elements may be hydrophobic or hydrophilic. In an embodiment, the enrobeable elements may be treated to be made hydrophobic. In an embodiment, the enrobeable elements may be treated to become hydrophilic.

The constituent fibers of the heterogeneous mass may be comprised of polymers such as polyethylene, polypropylene, polyester, and blends thereof. The fibers may be spunbound fibers. The fibers may be meltblown fibers or nano-fibers. The fibers may comprise cellulose, rayon, cotton, or other natural materials or blends of polymer and natural materials. The fibers may also comprise a super absorbent material such as polyacrylate or any combination of suitable materials. The fibers may be monocomponent, bicomponent, and/or biconstituent, non-round (e.g., capillary channel fibers), and may have major cross-sectional dimensions (e.g., diameter for round fibers) ranging from 0.1-500 microns. The constituent fibers of the nonwoven precursor web may also be a mixture of different fiber types, differing in such features as chemistry (e.g. polyethylene and polypropylene), components (mono- and bi-), denier (micro denier and >20 denier), shape (i.e. capillary and round) and the like. The constituent fibers may range from about 0.1 denier to about 100 denier.

In one aspect, known absorbent web materials in an as-made can be considered as being homogeneous throughout. Being homogeneous, the fluid handling properties of the absorbent web material are not location dependent, but are substantially uniform at any area of the web. Homogeneity can be characterized by density, basis weight, for example, such that the density or basis weight of any particular part of the web is substantially the same as an average density or basis weight for the web. By the apparatus and method of the present invention, homogeneous fibrous absorbent web materials are modified such that they are no longer homogeneous, but are heterogeneous, such that the fluid handling properties of the web material are location dependent. Therefore, for the heterogeneous absorbent materials of the present invention, at discrete locations the density or basis weight of the web may be substantially different than the average density or basis weight for the web. The heterogeneous nature of the absorbent web of the present invention permits the negative aspects of either of permeability or capillarity to be minimized by rendering discrete portions highly permeable and other discrete portions to have high capillarity. Likewise, the tradeoff between permeability and capillarity is managed such that delivering relatively higher permeability can be accomplished without a decrease in capillarity.

In an embodiment, the heterogeneous mass may also include superabsorbent material that imbibe fluids and form hydrogels. These materials are typically capable of absorbing large quantities of body fluids and retaining them under moderate pressures. The heterogeneous mass can include such materials dispersed in a suitable carrier such as cellulose fibers in the form of fluff or stiffened fibers.

In an embodiment, the heterogeneous mass may include thermoplastic particulates or fibers. The materials, and in particular thermoplastic fibers, can be made from a variety of thermoplastic polymers including polyolefins such as polyethylene (e.g., PULPEX.RTM.) and polypropylene, polyesters, copolyesters, and copolymers of any of the foregoing.

Depending upon the desired characteristics, suitable thermoplastic materials include hydrophobic fibers that have been made hydrophilic, such as surfactant-treated or silica-treated thermoplastic fibers derived from, for example, polyolefins such as polyethylene or polypropylene, polyacrylics, polyamides, polystyrenes, and the like. The surface of the hydrophobic thermoplastic fiber can be rendered hydrophilic by treatment with a surfactant, such as a nonionic or anionic surfactant, e.g., by spraying the fiber with a surfactant, by dipping the fiber into a surfactant or by including the surfactant as part of the polymer melt in producing the thermoplastic fiber. Upon melting and resolidification, the surfactant will tend to remain at the surfaces of the thermoplastic fiber. Suitable surfactants include nonionic surfactants such as Brij 76 manufactured by ICI Americas, Inc. of Wilmington, Del., and various surfactants sold under the Pegosperse.RTM. trademark by Glyco Chemical, Inc. of Greenwich, Conn. Besides nonionic surfactants, anionic surfactants can also be used. These surfactants can be applied to the thermoplastic fibers at levels of, for example, from about 0.2 to about 1 g. per sq. of centimeter of thermoplastic fiber.

Suitable thermoplastic fibers can be made from a single polymer (monocomponent fibers), or can be made from more than one polymer (e.g., bicomponent fibers). The polymer comprising the sheath often melts at a different, typically lower, temperature than the polymer comprising the core. As a result, these bicomponent fibers provide thermal bonding due to melting of the sheath polymer, while retaining the desirable strength characteristics of the core polymer.

Suitable bicomponent fibers for use in the present invention can include sheath/core fibers having the following polymer combinations: polyethylene/polypropylene, polyethylvinyl acetate/polypropylene, polyethylene/polyester, polypropylene/polyester, copolyester/polyester, and the like. Particularly suitable bicomponent thermoplastic fibers for use herein are those having a polypropylene or polyester core, and a lower melting copolyester, polyethylvinyl acetate or polyethylene sheath (e.g., DANAKLON.RTM., CELBOND.RTM. or CHISSO.RTM. bicomponent fibers). These bicomponent fibers can be concentric or eccentric. As used herein, the terms "concentric" and "eccentric" refer to whether the sheath has a thickness that is even, or uneven, through the cross-sectional area of the bicomponent fiber. Eccentric bicomponent fibers can be desirable in providing more compressive strength at lower fiber thicknesses. Suitable bicomponent fibers for use herein can be either uncrimped (i.e. unbent) or crimped (i.e. bent). Bicomponent fibers can be crimped by typical textile means such as, for example, a stuffer box method or the gear crimp method to achieve a predominantly two-dimensional or "flat" crimp.

The length of bicomponent fibers may vary depending upon the particular properties desired for the fibers and the web formation process. Typically, in an airlaid web, these thermoplastic fibers have a length from about 2mm to about 12mm long such as, for example, from about 2.5mm to about 7.5mm long, and from about 3.0mm to about 6.0mm long. Nonwoven fibers may be between 5 mm long and 75 mm long if used in a carded non-woven, such as, for example, 10 mm long, 15 mm long, 20 mm long, 25 mm long, 30 mm long, 35 mm long, 40 mm long, 45 mm long, 50 mm long, 55 mm long, 60 mm long, 65 mm long, or 70 mm long. In a spunbond process the fibers may be continuous not discrete. The properties-of these thermoplastic fibers may also be adjusted by varying the diameter (caliper) of the fibers. The diameter of these thermoplastic fibers is typically defined in terms of either denier (grams per 9000 meters) or decitex (grams per 10,000 meters). Suitable bicomponent thermoplastic fibers as used in an airlaid making machine may have a decitex in the range from about 1.0 to about 20 such as, for example, from about 1.4 to about 10, and from about 1.7 to about 7 decitex.

The compressive modulus of these thermoplastic materials, and especially that of the thermoplastic fibers, can also be important. The compressive modulus of thermoplastic fibers is affected not only by their length and diameter, but also by the composition and properties of the polymer or polymers from which they are made, the shape and configuration of the fibers (e.g., concentric or eccentric, crimped or uncrimped), and like factors. Differences in the compressive modulus of these thermoplastic fibers can be used to alter the properties, and especially the density characteristics, of the respective thermally bonded fibrous matrix.

The heterogeneous mass can also include synthetic fibers that typically do not function as binder fibers but alter the mechanical properties of the fibrous webs. Synthetic fibers include cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. These might include, for example, polyester fibers such as polyethylene terephthalate (e.g., DACRON.RTM. and KODEL.RTM.), high melting crimped polyester fibers (e.g., KODEL.RTM. 431 made by Eastman Chemical Co.) hydrophilic nylon (HYDROFIL.RTM.), and the like. Suitable fibers can also hydrophilized hydrophobic fibers, such as surfactant-treated or silica-treated thermoplastic fibers derived from, for example, polyolefins such as polyethylene or polypropylene, polyacrylics, polyamides, polystyrenes, polyurethanes and the like. In the case of nonbonding thermoplastic fibers, their length can vary depending upon the particular properties desired for these fibers. Typically they have a length from about 30 to 75 mm, preferably from about 9 to about 15 mm. Suitable nonbonding thermoplastic fibers can have a decitex in the range of about 1.5 to about 35 decitex, such as from about 14 to about 20 decitex.

However structured, the total absorbent capacity of the heterogeneous mass containing foam pieces should be compatible with the design loading and the intended use of the mass. For example, when used in an absorbent article, the size and absorbent capacity of the heterogeneous mass may be varied to accommodate different uses such as incontinence pads, pantiliners, regular sanitary napkins, or overnight sanitary napkins.

The heterogeneous mass can also include other optional components sometimes used in absorbent webs. For example, a reinforcing scrim can be positioned within the respective layers, or between the respective layers, of the heterogeneous mass.

The heterogeneous mass comprising open-cell foam pieces produced from the present invention may be used as an absorbent core or a portion of an absorbent core in absorbent articles, such as feminine hygiene articles, for example pads, pantiliners, and tampons; disposable diapers; incontinence articles, for example pads, adult diapers; homecare articles, for example wipes, pads, towels; and beauty care articles, for example pads, wipes, and skin care articles, such as used for pore cleaning.

The heterogeneous mass layer may be formed or cut to a shape, the outer edges of which define a periphery.

In an embodiment, the open-cell foam pieces are in the form of stripes. The stripes may be formed during the formation of the heterogeneous mass or by formation means after polymerization. The stripes may run along the longitudinal length of the heterogeneous mass layer, along the lateral length of the heterogeneous mass layer, or a combination of both the longitudinal length and the lateral length. The stripes may run along a diagonal to either the longitudinal length or the lateral length of the heterogeneous mass layer. The stripes are separated by canals.

Formation means known for deforming a generally planar fibrous web into a three-dimensional structure are utilized in the present invention to modify as-made absorbent materials into absorbent materials having relatively higher permeability without a significant corresponding decrease in capillary pressure. Formation means may comprise a pair of inter-meshing rolls, typically steel rolls having inter-engaging ridges or teeth and grooves. However, it is contemplated that other means for achieving formation can be utilized, such as the deforming roller and cord arrangement disclosed in US 2005/0140057 published June 30, 2005. Therefore, all disclosure of a pair of rolls herein is considered equivalent to a roll and cord, and a claimed arrangement reciting two inter-meshing rolls is considered equivalent to an inter-meshing roll and cord where a cord functions as the ridges of a mating inter-engaging roll. In one embodiment, the pair of intermeshing rolls of the instant invention can be considered as equivalent to a roll and an inter-meshing element, wherein the inter-meshing element can be another roll, a cord, a plurality of cords, a belt, a pliable web, or straps. Likewise, other known formation technologies, such as creping, necking/consolidation, corrugating, embossing, button break, hot pin punching, and the like are believed to be able to produce absorbent materials having some degree of relatively higher permeability without a significant corresponding decrease in capillary pressure. Formation means utilizing rolls include "ring rolling", a "SELF" or "SELF'ing" process, in which SELF stands for Structural Elastic Like Film, as "micro-SELF", and "rotary knife aperturing" (RKA); as described in US Patent No. 7,935,207 Zhao et al., granted May 3, 2011.

In an embodiment, the absorbent core structure has an absorbent layer that comprises superabsorbent particles. The superabsorbent particles may be on a substrate or within a nonwoven layer.. The absorbent layer may additionally comprise a thermoplastic. In an embodiment, the absorbent core layer may comprise of any layer or combination of layers as described in US 8,263,820; US 8,124,827; US patent publication no. 2010-0228209 A1; or US patent publication no. 2010-0262104 A1.

The substrate of the absorbent layer may comprise a fibrous material. The fibrous material may comprise rayon, cellulose, viscose, naturally occurring fibers, and any other fiber known to one of skill in the art including all the materials listed above or incorporated herein for the enrobeable element which are fibrous. The fibrous material may be substantially free of cellulose fibers. The substrate layer 100 can also have a basis weight from 25 g/m² to 120 g/m², or from 35 g/m² to 90 g/m². The substrate of the absorbent layer may comprise a fibrous material comprising rayon.

The thermoplastic material may comprise, in its entirety, a single thermoplastic polymer or a blend of thermoplastic polymers, having a softening point, as determined by the ASTM Method D-36-95 "Ring and Ball", in the range between 50°C and 300°C, or alternatively the thermoplastic composition may be a hot melt adhesive comprising at least one thermoplastic polymer in combination with other thermoplastic diluents such as tackifying resins, plasticizers and additives such as antioxidants.

The substrate may comprise thermoplastic material. The thermoplastic polymer can have typically a molecular weight (Mw) of more than 10,000 and a glass transition temperature (Tg) usually below room temperature. Typical concentrations of the polymer in a hot melt are in the range of 20 - 40 % by weight. A wide variety of thermoplastic polymers can be suitable for use in the present invention. Such thermoplastic polymers can be typically water insensitive. Exemplary polymers can be (styrenic) block copolymers including A-B-A triblock structures, A-B diblock structures and (A-B)n radial block copolymer structures wherein the A blocks can be non-elastomeric polymer blocks, typically comprising polystyrene, and the B blocks can be unsaturated conjugated diene or (partly) hydrogenated versions of such. The B block can be typically isoprene, butadiene, ethylene/butylene (hydrogenated butadiene), ethylene/propylene (hydrogenated isoprene), and mixtures thereof.

Other suitable thermoplastic polymers that may be employed are metallocene polyolefins, which are ethylene polymers prepared using single-site or metallocene catalysts. Therein, at least one co-monomer can be polymerized with ethylene to make a copolymer, terpolymer or higher order polymer. Also applicable can be amorphous polyolefins or amorphous polyalphaolefins (APAO) which are homopolymers, copolymers or terpolymers of C2 to C8 alphaolefins.

The resin can typically have a Mw below 5,000 and a Tg usually above room temperature, typical concentrations of the resin in a hot melt can be in the range of 30 - 60 %. The plasticizer has a low Mw of typically less than 1,000 and a Tg below room temperature, a typical concentration is 0 -15%.

The thermoplastic material, typically a hotmelt adhesive, can be present in the form of fibers throughout the core, being provided with known means, i.e. the adhesive can be fiberized. Typically, the fibers can have an average thickness of 1 - 100 micrometer and an average length of 5 mm to 50 cm. In particular the layer of thermoplastic material, typically e.g. a hot melt adhesive, can be provided such as to comprise a net-like structure.

To improve the adhesiveness of the thermoplastic material to the substrate layer or to any other layer, in particular any other non-woven layer, such layers may be pre-treated with an auxiliary adhesive.

An absorbent core layer may have absorbent polymer material. Without wishing to be bound by theory it is believed that such material, even in the swollen state, i.e. when liquid has been absorbed, does not substantially obstruct the liquid flow throughout the material, particularly when further the permeability of said material, as expressed by the saline flow conductivity of the absorbent polymer material, is greater than 10, 20, 25, 30, 40, 50, 100, or 200 SFC- units, where 1 SFC unit is 1 x 10⁻⁷ (cm³ x s) / g. Saline flow conductivity is a parameter well recognized in the art and is to be measured in accordance with the test disclosed in EP 752 892 B.

This layer of absorbent polymer material can be typically a non-uniform layer, and comprises a first surface and a second surface, wherein by "non-uniform" it is meant that the absorbent polymer material is distributed over a substrate with non-uniform basis weight. Conversely, the second surface of the non-uniform layer of absorbent polymer material is in at least partial contact with the first surface of the substrate layer. According to an embodiment of the present invention, the non-uniform layer of absorbent polymer material can be a discontinuous layer that is a layer typically comprising openings, i.e. areas substantially free of absorbent polymer material, which in certain embodiments can be typically completely surrounded by areas comprising absorbent polymer material.

Suitable absorbent polymer materials for use in the invention can comprise a substantially water-insoluble, slightly crosslinked, partially neutralized, polymeric gelling material. This material forms a hydrogel upon contact with water. Such polymer materials can be prepared from polymerizable, unsaturated, acid-containing monomers. Suitable unsaturated acidic monomers for use in preparing the polymeric absorbent gelling material used in this invention include those listed in U.S. Pat. No. 4,654,039 (Brandt et al), issued Mar. 31, 1987, and reissued as RE 32,649 on Apr. 19, 1988.

Preferred monomers include acrylic acid, methacrylic acid, and 2-acrylamido-2-methyl propane sulfonic acid. Acrylic acid itself is especially preferred for preparation of the polymeric gelling material. The polymeric component formed from the unsaturated, acid-containing monomers can be grafted onto other types of polymer moieties such as starch or cellulose. Polyacrylate grafted starch materials of this type are especially preferred. Preferred polymeric absorbent gelling materials that can be prepared from conventional types of monomers include hydrolyzed acrylonitrile grafted starch, polyacrylate grafted starch, polyacrylates, maleic anhydride-based copolymers and combinations thereof.

The absorbent article comprises a liquid pervious topsheet. The topsheet suitable for use herein can comprise wovens, non-wovens, and/or three-dimensional webs of a liquid impermeable polymeric film comprising liquid permeable apertures. The topsheet for use herein can be a single layer or may have a multiplicity of layers. For example, the wearer-facing and contacting surface can be provided by a film material having apertures which are provided to facilitate liquid transport from the wearer facing surface towards the absorbent structure. Such liquid permeable, apertured films are well known in the art. They provide a resilient three-dimensional fibre-like structure. Such films have been disclosed in detail for example in US 3929135, US 4151240, US 4319868, US 4324426, US 4343314, US 4591523, US 4609518, US 4629643, US 4695422 or WO 96/00548.

The absorbent layers may be combined using bonds, a bonding layer, adhesives, or combinations thereof. The absorbent core structure may be attached to the topsheet, the backsheet, or both the topsheet and backsheet using bonds, a bonding layer, adhesives, or combinations thereof. Adhesives may be placed in any suitable pattern, such as, for example, lines, spirals, points, circles, squares, or any other suitable pattern. Bonds may be placed in any suitable pattern, such as, for example, lines, spirals, points, circles, squares, or any other suitable pattern.

The absorbent layers may be combined using an intermediate layer between the two layers. The intermediate layer may comprise a tissue, a nonwoven, a film, or combinations thereof. The intermediate layer may have a permeability greater than the 200 Darcy, 400 Darcy, 600 Darcy, 800 Darcy, or 1,000 Darcy.

The core structure is a two layer core structure. The upper layer is a heterogeneous mass comprising open-cell foam. The open-cell foam may comprise canals along the longitudinal length of the core. The lower layer comprises a substrate layer with superabsorbent polymer placed on top of the substrate. The substrate and superabsorbent polymer may be coated by a thermoplastic. The two layer core structure may be combined with other layers provided that the additional layers are placed below the two layer core structure.

The canals within the upper layer of the two layer core structure may end before the edge of the core. The canals may be continuous or discontinuous. The canals may between 0.1 inches and 3 inches from each end of the core, such as, for example, 0.2 inches, 0.25 inches, 0.3 inches, 0.35 inches, 0.4 inches, 0.45 inches, or 0.5 inches. Without being bound by theory, Applicants have found that the canals within the upper layer may carry the fluid away from the insult area making it accessible to portions of the lower core that would otherwise not see the fluid insult. The canals rapidly disperse fluid away from the loading or insult zone and utilize the void volume leading to faster acquisition times. At the same time, the canals provide high suction walls that provide active wicking of the fluids.

The canals may be spaced between 0.1 mm and 5 mm apart, such as for example, between 0.5 mm and 4 mm, or between 1 mm and 3 mm apart. In an embodiment, the canals are spaced such that they are parallel with each other and from 30% to 100%, or from 40% to 95%, or from 50% to 90%, or from 60% to 85% of the length of the longitudinal dimension, transverse dimension, lateral dimension, or a diagonal dimension of the heterogeneous mass top core structure. The canals may parallel a longitudinal axis, a transverse axis, a lateral axis, or a diagonal axis of the heterogeneous mass top core structure. In an embodiment, the canals are in the form of sinusoidal waves versus straight lines. In an embodiment, the canals are in the form of any suitable geometric design such as, for example, spirals, swirls, lines, squares, waves, etc.

Without being bound by theory, Applicants have found that the two layer core structure described above allows for high capillarity suction while maintaining high permeability across an entire surface while maintaining a controlled ratio of permeability down through the canals relative to the capillarity of fluid through the canal walls. The ability to increase canal height and longitudinal flow rate creates a unique swelling phenomenon. Specifically, for a given insult amount, the overall swelling in the insult area associated with the AGM layer, the substrate layer, or a combined AGM layer plus one or more substrate layers is lower and more even along a product than ever before because the canals are so effective and transporting fluid away from the insult area. A series of parallel canals creates a structure that is unique relative to dimensional flexibility.

In an embodiment, the absorbent core comprises of at least two layers. The top core layer comprises a heterogeneous mass containing a HIPE foam intermixed with a nonwoven web. The heterogeneous mass contains one or more canals of high capillarity and one or more canals of high permeability. The canals of high capillarity contain a high density of HIPE and nonwoven web and wherein the canals of high permeability contain a low density of HIPE and nonwoven web. The lower core level comprises a substrate layer with superabsorbent polymer containing greater than 50%, of a superabsorbent polymer and less than 30% of cellulose.

Without being bound by theory, Applicants have found that, when used as an absorbent core in an absorbent article, the canal walls of the heterogeneous mass comprising open-cell foam layer create a unique dynamic volume canal and fluid transport system based on liquid insults. More specifically, the canal walls vertically swell after liquid insults so that the canals are deeper and contain more volume capacity for subsequent insults. The canals do not significantly swell in the X-Y directions. The canals also maintain a relatively constant rate of fluid flow along the length of the canals due to the capillarity of the canal walls.

The two layer core structure allows for improved acquisition times for subsequent gushes.

Tables 1 and 2 show the measured results for the parameters mentioned in the present application for a number of products. The products indicated as "Brand X" are commercial products and are selected among commonly available Menstrual and incontinence products.

Prototype products 0 and A to I are prototypes of which B-I are according to the invention.

In all prototypes wherein foam is present the foam used is a HIPE prepared from a 27:1 water in oil emulsion with the same composition as used in the lower layer of the Infinity in-market product.
Brand A - Foam product = Always Infinity F3 size
Brand S - Maxi Product = Stayfree Maxi Super Pad
Brand K - Ultra Product= U By Kotex Overnight Ultra Thins
Brand A - Ultra Product= Always Ultra Thin
Brand P Medium Absorbency= Poise Maximum Absorbency Long size
Brand A Medium Absorbency= Always Discrete Pads Maximum size
Brand T Medium Absorbency= Tena Serenity Heavy Long size
Brand P Medium Absorbency- New= Poise Thin Shaped Pads Size 3

### Prototype 0 - (not according to the invention)

This product is based on the in market product referred to as "Brand A - Medium Absorbency", namely Always Discrete pads maximum size. The absorbent core system of the market products has been carefully removed and replaced with a new absorbent core, the product has then been resealed. The new core system is formed by an AGM particle layer sandwiched between a top nonwoven layer facing the body, and a bottom nonwoven layer facing the panty. The AGM layer is immobilized on both sides by adhesives.

The top nonwoven layer is a 75gsm Spunlace manufactured by Sandler AG (Germany) under the brand name Sawasoft and is composed of the fibers: 45% Viscose Rayon (1.3DTex, 50mm); 40 % BiCo Fiber (PE/PET, 2.2 Dtex, 38-40mm); 15 % HollowSpiral PET (10 Dtex, 38-40mm).

The AGM layer contains 273gsm of Shokobai AGM manufactured under the trade name of Aqualic CA L-700.

The AGM particle layer is immobilized on the body facing side by meltblown adhesive layer applied in the form of microfibers with a basis weight of 10gsm and manufactured by HB Fuller Adhesives (USA) under the manufactures Code NW1151 ZP. On the panty facing side AGM is immobilized by is a slot coated adhesive layer applied with a basis weight of 6.0gsm and manufactured by HB Fuller Adhesives (USA) under the manufactures Code HL 1358LO-F ZP.

The bottom nonwoven layer is a 345gsm Airlaid material manufactured by Glatfelter GmbH (Germany) under the manufactures Code MH345.231

This Absorbent core has a rectangular shape with an overall width of 288mm long and 69mm wide. The AGM pattern is also 288mm long but only 61 mm wide so it is contained away from the edges of the core system to avoid side leakage.

Prototype A (not according to the invention) is based on the structure of the "Brand A - Foam Product", namely Always Infinity Heavy Flow (F3). The absorbent core system of the market products has been carefully removed and replaced with an absorbent element according to the present invention, the product has then been resealed.

The absorbent element consists of a core structure formed by a layer of heterogeneous mass comprising HIPE open cell foam layer enrobing the fibers of two nonwoven layers sandwiching it. The emulsion is extruded onto a carrier nonwoven which is a 60gsm acquisition layer 3 material manufactured by Fitesa - green Bay (USA) with the Product Code 9360770370, the emulsion enrobes the fibers of the nonwoven. This layer is positioned towards the panty side of the product. Before polymerization a second nonwoven is applied onto the exposed HIPE surface thus creating a second enrobed layer. The second nonwoven is a 55gsm Spunlace nonwoven manufactured by Sandler AG (Germany) under the brand name Sawasoft and is composed of the fibers: 45% Viscose Rayon (1.3DTex, 50mm); 40 % BiCo Fiber (PE/PET, 2.2 Dtex, 38-40mm); 15 % HollowSpiral PET (10 Dtex, 38-40mm).

Prototype B is based on the structure of the "Brand A - Medium Absorbency", namely Always Discrete pads maximum size. The absorbent core system of the market products has been carefully removed and replaced with an absorbent element according to the present invention, the product has then been resealed.

The absorbent element consists of a core structure formed by two layers:
The first layer is a heterogeneous mass comprising HIPE open cell foam pieces enrobing the fibers of a nonwoven, the second layer is a layer of AGM immobilized between two nonwoven substrates with fiberized hot melt glue.

The first layer is prepared by extruding the a foam precursor 27:1 HIPE emulsion as a uniform layer at a basis weight of 150 gsm onto the substrate nonwoven which is a 43gsm acquisition layer material produced by Fitesa (USA) with product code 9343789370. The emulsion enrobes the fibers of the nonwoven before being polymerized to an open celled foam having an expanded pore size distribution of 2-50 microns. The resulting material is mechanically treated with intermeshing roll as described so to open the foam layer and form discrete canals thus forming parallel stripes of foam in the longitudinal direction of the product separated by canals with canal openings of width 2mm and height of 1mm. In total 17 canals are formed in web having a width of 70mm and a length of 270mm.

The second layer is prepared as a substrate plus superabsorbent polymer layer and is a laminate where the top (body facing) sub-layer is a 55gsm spunlace nonwoven manufactured by Sandler (Germany) under the brand name Sawasoft and is composed of the fibers: 45% Viscose Rayon (1.3DTex, 50mm); 40 % BiCo Fiber (PE/PET, 2.2 Dtex, 38-40mm); 15 % HollowSpiral PET (10 Dtex, 38-40mm). The AGM sub-layer contains 315gsm of Shokobai AGM manufactured under the trade name of Aqualic CA L-700. The bottom (panty facing) sub-layer bottom is a standard 10gsm polypropylene nonwoven spunbond material manufactured Fibertex (Denmark) used simply to contain the AGM particles. The AGM particles are immobilized on the top side by a meltblown adhesive sub-layer applied in the form of microfibers with a basis weight of 10gsm and manufactured by HB Fuller Adhesives (USA) under the manufactures Code NW1151 ZP. On the bottom facing side AGM is immobilized by a further slot coated adhesive sub-layer applied with a basis weight of 6.0gsm where the adhesive is manufactured by HB Fuller Adhesives (USA) under the manufactures Code HL 1358LO-F ZP.

This second layer has a rectangular shape with an overall width of 288mm long and 69mm wide. The AGM pattern is also 288mm long but only 61 mm wide so it is contained away from the edges of the core system to avoid side leakage.

The first layer is positioned closer to the body and is oriented so that the side with channels is oriented toward the panty.

Prototype C is based on prototype B wherein the nonwoven of the first layer is replaced with a 55gsm Spunlace nonwoven manufactured by Sandler AG (Germany) under the brand name Sawasoft and is composed of the fibers: 45% Viscose Rayon (1.3DTex, 50mm); 40 % BiCo Fiber (PE/PET, 2.2 Dtex, 38-40mm); 15 % HollowSpiral PET (10 Dtex, 38-40mm).

Prototype D is based on prototype C wherein the expanded pore size distribution of the foam is 2-30 microns, wherein the canal openings is 1.5mm instead of 2mm and wherein the canals are 22 over the 70mm web width. In the second layer the top sub-layer is 75gsm Spunlace nonwoven manufactured by Sandler AG (Germany) under the brand name Sawasoft and is composed of the fibers: 35% Galaxy Tri-lobal Rayon (3.3DTex, 38mm); 40 % PolyPropylene Fiber (6.7 Dtex, 38-40mm); 25 % HollowSpiral PET (10 Dtex, 38-40mm) and the bottom sub-layer is a 65gsm Airlaid material manufactured by Glatfelter GmbH (Germany) under the manufactures Code VH065.103 used to contain the AGM particles and add additional void volume to the core system.

Prototype E is based on prototype D wherein the nonwoven of the first layer is a 60gsm acquisition layer 3 material manufactured by Fitesa - green Bay (USA) with the Product Code 9360770370.

Prototype F is based on prototype E wherein the nonwoven of the first layer is a 55gsm Spunlace nonwoven manufactured by Sandler AG (Germany) under the brand name Sawasoft and is composed of the fibers: 45% Viscose Rayon (1.3DTex, 50mm); 40 % BiCo Fiber (PE/PET, 2.2 Dtex, 38-40mm); 15 % HollowSpiral PET (10 Dtex, 38-40mm) and wherein in the second layer the bottom sub-layer is a standard 10gsm polypropylene nonwoven spunbond material manufactured Fibertex (Denmark) used simply to contain the AGM particles.

Prototype G is based on prototype F where the nonwoven material of the first layer is a 60gsm Acquisition layer 3 material manufactured by Fitesa - green Bay (USA) with the Product Code 9360770370, and wherein in the second layer the bottom sub-layer is a standard 10gsm polypropylene nonwoven spunbond material manufactured Fibertex (Denmark) used simply to contain the AGM particles.

Prototype H is based on prototype G wherein, in the second layer, the top sub-layer is a 30gsm Spunlace nonwoven manufactured by Suominen (Finland) under the brand name Fibrella Spunlace Product Code F2000 (67% 2.2 Dtex Viscose, 33% 3DTex PET) and the bottom sub-layer is a 65gsm Airlaid material manufactured by Glatfelter GmbH (Germany) under the manufactures Code VH065.103 used to contain the AGM particles and add additional void volume to the core system.

Prototype I is based on prototype H wherein, in the second layer, the bottom sub-layer is a standard 10gsm polypropylene nonwoven spunbond material manufactured Fibertex (Denmark) used simply to contain the AGM particles.

**Table 1. Physical Properties of Products**

| **Menstrual Products** | **Dry Caliper** mm | **Dry Peak Stiffness** Newton (N) | **Acq. rate (SABAP)** ml/sec | **% Caliper Expansion** @ 5min | **% Caliper Expansion** @ 1 min | **Rewet (SABAP)** grams |
|---|---|---|---|---|---|---|
| Brand A - Foam Product | 2.61 | 1.40 | 0.04 | 54% | 49% | 0.41 |
| Brand S - Maxi Product | 10.00 | 12.60 | 0.12 | 7% | 2% | 0.13 |
| Brand K - Ultra Product | 3.59 | 3.90 | 0.06 | 15% | 6% | 0.28 |
| Brand A - Ultra Product | 1.99 | 1.30 | 0.05 | 46% | 21% | 0.31 |
| Prototype Product A | 3.13 | 3.90 | 0.04 | 30% | 27% | 0.27 |
| **Incontinence Products** - **Pads** | | | | | | |
| Brand P Medium Absorbency | 11.25 | 49.60 | 3.02 | 110% | 60% | 0.03 |
| Brand A Medium Absorbency | 5.35 | 39.70 | 0.77 | 112% | 53% | 0.03 |
| Brand T Medium Absorbency | 10.09 | 34.00 | 3.16 | 111% | 44% | 0.04 |
| Prototype Product 0 | 4.80 | 6.30 | 0.78 | 258% | 140% | 0.03 |
| Brand P Medium Absorbency- New | 5.15 | 27.00 | 0.69 | 257% | 137% | 0.03 |
| **Incontinence Products** - **Pants/Diapers** | | | | | | |
| Brand A - Adult | 7.81 | 10.70 | 4.19 | 117% | 58% | 0.03 |
| Brand P - Baby | 6.90 | 13.30 | 2.01 | 99% | 23% | 0.02 |
| Brand H - Baby | 5.25 | 7.70 | 2.25 | 227% | 93% | 0.02 |
| **Inventive Prototypes** | | | | | | |
| Prototype Product B | 3.60 | 1.30 | 2.33 | 349% | 220% | 0.09 |
| Prototype Product C | 3.55 | 1.23 | 2.10 | 298% | 201% | 0.08 |
| Prototype Product D | 4.39 | 2.45 | 0.73 | 202% | 123% | 0.08 |
| Prototype Product E | 5.65 | 2.94 | 1.58 | 244% | 138% | 0.10 |
| Prototype Product F | 3.73 | 1.77 | 0.62 | 214% | 155% | 0.08 |
| Prototype Product G | 4.99 | 2.16 | 1.45 | 292% | 183% | 0.08 |
| Prototype Product H | 5.28 | 1.67 | 0.86 | 229% | 165% | 0.08 |
| Prototype Product I | 4.72 | 1.47 | 0.88 | 248% | 192% | 0.08 |

**Table 2. Physical Property Relationships**

| **Menstrual Product** | **Acq. Rate/ Dry Peak Stiffness** (ml/Ns) | **Dry Caliper/Dry Peak Stiffness** (mm)/N | **Dry Caliper* Acq. Rate/Dry Peak Stiffness** mm*ml/sN | **% Caliper Expansion/Dry Peak Stiffness** (%/N ) | **Dry Caliper/** % **caliper change at** (mm*sec/mL) At 60 sec. | **AcqRate/ Peak Force** * **Rewet (mL/sec/N.g)** |
|---|---|---|---|---|---|---|
| Brand A - Foam Product | 0.03 | 1.86 | 0.07 | 39% | 5.33 | 0.07 |
| Brand S - Maxi Product | 0.01 | 0.79 | 0.10 | 1% | 500.00 | 0.08 |
| Brand K - Ultra Product | 0.02 | 0.92 | 0.06 | 4% | 65.27 | 0.07 |
| Brand A - Ultra Product | 0.04 | 1.53 | 0.08 | 35% | 9.48 | 0.13 |
| Prototype Product A | 0.01 | 0.80 | 0.03 | 8% | 11.59 | 0.04 |
| **Incontinence Products** - **Pads** | | | | | | |
| Brand P - Medium Absorbency | 0.06 | 0.23 | 0.68 | 2% | 18.75 | 1.82 |
| Brand A - Medium Absorbency | 0.02 | 0.13 | 0.10 | 3% | 10.09 | 0.63 |
| Brand T - Medium Absorbency | 0.09 | 0.30 | 0.94 | 3% | 22.93 | 2.25 |
| Brand A - Medium Absorbency - New | 0.12 | 0.76 | 0.59 | 41% | 3.43 | 4.80 |
| Brand P - Medium Absorbency - New | 0.03 | 0.19 | 0.13 | 10% | 3.76 | 1.00 |
| **Incontinence Products** - **Pants/Diapers** | | | | | | |
| Brand A - Adult | 0.39 | 0.73 | 3.06 | 11% | 13.47 | 13.00 |
| Brand P - Baby | 0.15 | 0.52 | 1.04 | 7% | 30.00 | 7.50 |
| Brand H - Baby | 0.29 | 0.68 | 1.53 | 29% | 5.65 | 14.50 |
| **Inventive Prototypes** | | | | | | |
| Prototype Product B | 1.79 | 2.76 | 6.43 | 268% | 1.64 | 19.89 |
| Prototype Product C | 1.71 | 2.90 | 6.08 | 243% | 1.77 | 21.41 |
| Prototype Product D | 0.30 | 1.79 | 1.31 | 83% | 3.57 | 3.73 |
| Prototype Product E | 0.54 | 1.92 | 3.04 | 83% | 4.09 | 5.66 |
| Prototype Product F | 0.35 | 2.11 | 1.30 | 121% | 2.41 | 4.65 |
| Prototype Product G | 0.67 | 2.31 | 3.36 | 135% | 2.73 | 8.22 |
| Prototype Product H | 0.52 | 3.17 | 2.73 | 137% | 3.20 | 6.47 |
| Prototype Product I | 0.60 | 3.21 | 2.83 | 169% | 2.46 | 7.39 |

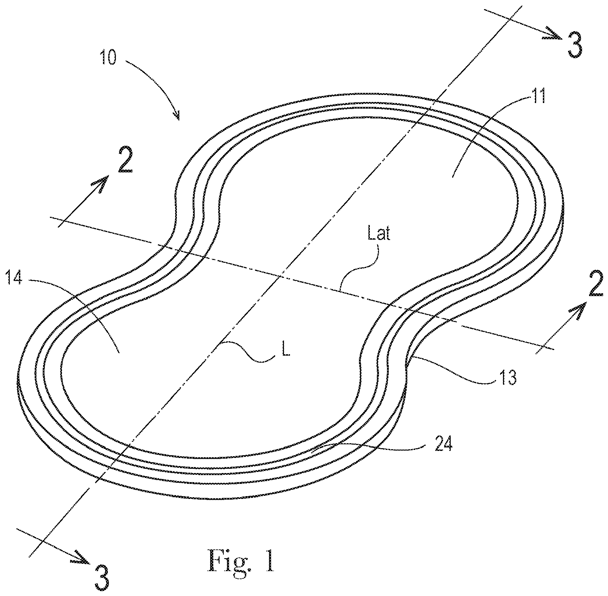

FIG. 1 a perspective view of one embodiment of a sanitary napkin. The illustrated sanitary napkin 10 has a body-facing upper side 11 that contacts the user's body during use. The opposite, garment-facing lower side 13 contacts the user's clothing during use.

A sanitary napkin 10 can have any shape known in the art for feminine hygiene articles, including the generally symmetric "hourglass" shape as shown in FIG. 1, as well as pear shapes, bicycle-seat shapes, trapezoidal shapes, wedge shapes or other shapes that have one end wider than the other. Sanitary napkins and pantyliners can also be provided with lateral extensions known in the art as "flaps" or "wings". Such extensions can serve a number of purposes, including, but not limited to, protecting the wearer's panties from soiling and keeping the sanitary napkin secured in place.

The upper side of a sanitary napkin generally has a liquid pervious topsheet 14. The lower side generally has a liquid impervious backsheet 16 that is joined with the topsheet 14 at the edges of the product. An absorbent core 18 is positioned between the topsheet 14 and the backsheet 16. A secondary topsheet may be provided at the top of the absorbent core 18, beneath the topsheet. The sanitary napkin 10 has a longitudinal axis (L) and a latitudinal axis (Lat).

The topsheet 14, the backsheet 16, and the absorbent core 18 can be assembled in a variety of well- known configurations, including so called "tube" products or side flap products, such as, for example, configurations are described generally in U.S. Pat. No. 4,950,264, "Thin, Flexible Sanitary Napkin" issued to Osborn on Aug. 21, 1990, U.S. Pat. No. 4,425,130, "Compound Sanitary Napkin" issued to DesMarais on Jan. 10, 1984; U.S. Pat. No. 4,321,924, "Bordered Disposable Absorbent Article" issued to Ahr on Mar. 30, 1982; U.S. Pat. No. 4,589,876, and "Shaped Sanitary Napkin With Flaps" issued to Van Tilburg on Aug. 18, 1987.

The backsheet 16 and the topsheet 14 can be secured together in a variety of ways. Adhesives manufactured by H. B. Fuller Company of St. Paul, Minn. under the designation HL-1258 or H-2031 have been found to be satisfactory. Alternatively, the topsheet 14 and the backsheet 16 can be joined to each other by heat bonding, pressure bonding, ultrasonic bonding, dynamic mechanical bonding, or a crimp seal. A fluid impermeable crimp seal 24 can resist lateral migration ("wicking") of fluid through the edges of the product, inhibiting side soiling of the wearer's undergarments.

As is typical for sanitary napkins and the like, the sanitary napkin 10 of the present invention can have panty-fastening adhesive disposed on the garment-facing side of backsheet 16. The panty-fastening adhesive can be any of known adhesives used in the art for this purpose, and can be covered prior to use by a release paper, as is well known in the art. If flaps or wings are present, panty fastening adhesive can be applied to the garment facing side so as to contact and adhere to the underside of the wearer's panties.

The backsheet may be used to prevent the fluids absorbed and contained in the absorbent structure from wetting materials that contact the absorbent article such as underpants, pants, pyjamas, undergarments, and shirts or jackets, thereby acting as a barrier to fluid transport. The backsheet according to an embodiment of the present invention can also allow the transfer of at least water vapour, or both water vapour and air through it.

Especially when the absorbent article finds utility as a sanitary napkin or panty liner, the absorbent article can be also provided with a panty fastening means, which provides means to attach the article to an undergarment, for example a panty fastening adhesive on the garment facing surface of the backsheet. Wings or side flaps meant to fold around the crotch edge of an undergarment can be also provided on the side edges of the napkin.

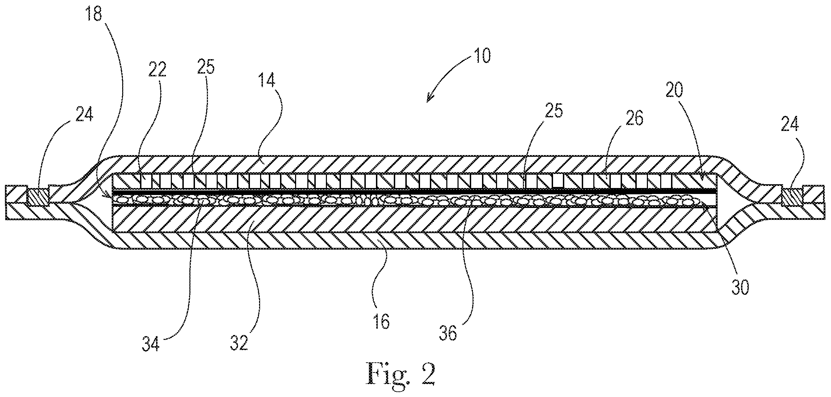

FIG. 2 is a cross-sectional view of the sanitary napkin 10 of Fig. 1, taken through line 2-2. As shown in the figure, the absorbent core 18 structure comprises of an upper layer 20 and a lower layer 30. The upper layer 20 is a heterogeneous mass 22 comprising open-cell foam pieces 25. The open-cell foam pieces 25 are in the form of stripes 26 that run along the longitudinal length of the absorbent article 10. The absorbent foam pieces 25 are separated by canals 28. The absorbent core 18 structure lower layer 30 comprises a substrate 32 with superabsorbent polymer 34 on top of the substrate 32. The substrate 32 and polymer 34 are coated with a thermoplastic adhesive 36.

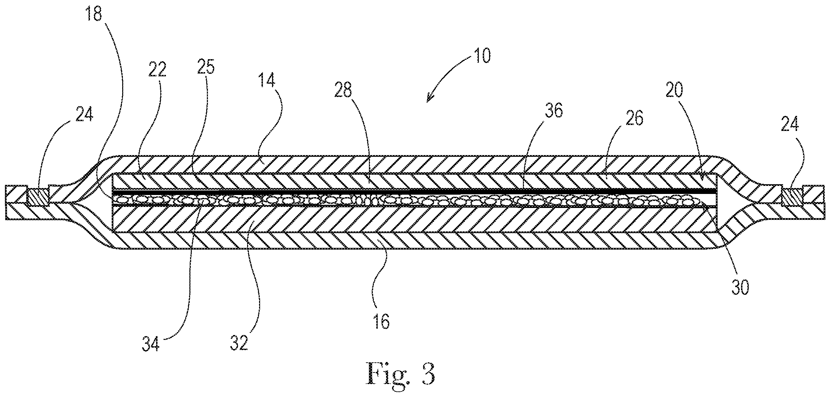

FIG. 3 is a cross-sectional view of the sanitary napkin 10 of Fig. 1, taken through line 3-3. As shown in the figure, the absorbent core 18 structure comprises of an upper layer 20 and a lower layer 30. The upper layer 20 is a heterogeneous mass 22 comprising open-cell foam pieces 25. The open-cell foam pieces 25 are in the form of stripes 26 that run along the longitudinal length of the absorbent article 10. The absorbent core 18 structure lower layer 30 comprises a substrate 32 with superabsorbent polymer 34 on top of the substrate 32. The substrate 32 and polymer 34 are coated with a thermoplastic adhesive 36.

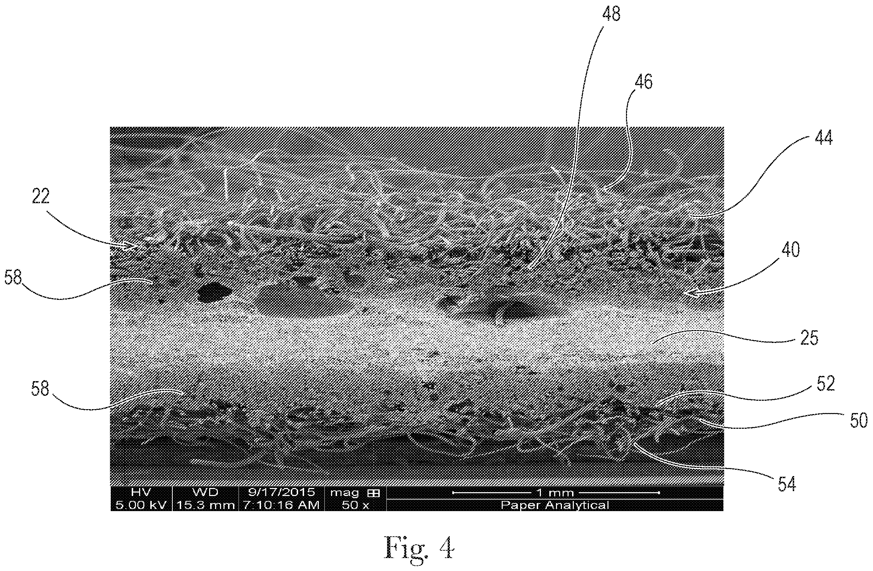

FIG. 4 is an SEM micrograph of a heterogeneous mass 22 prior to any formation means or forming of canals. As shown in FIG. 4, the absorbent stratum 40 is a heterogeneous mass 22 comprising a first planar nonwoven 44 having a first surface 46 and a second surface 48 and a second planar nonwoven 50 having a first surface 52 and a second surface 54. An open cell foam piece 25 enrobes a portion of the first planar nonwoven 44 and a portion of the second planar nonwoven 50. Specifically, the open cell foam piece 25 enrobes enrobeable elements 58 in both the second surface 48 of the first planar nonwoven 44 and the first surface 42 of the second planar nonwoven 50.

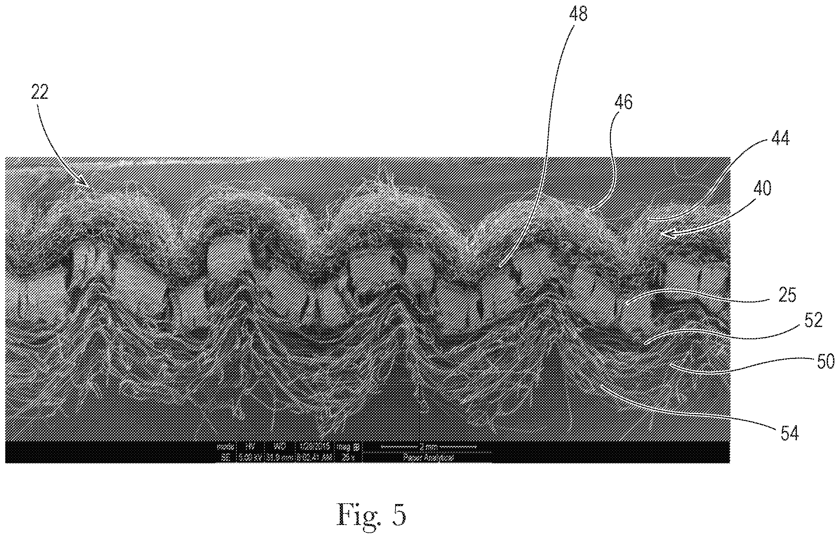

FIG. 5 is an SEM micrograph of a heterogeneous mass 22 after formation means or the forming of canals. As shown in FIG. 5, the absorbent stratum 40 is a heterogeneous mass 22 comprising a first planar nonwoven 44 having a first surface 46 and a second surface 48 and a second planar nonwoven 50 having a first surface 52 and a second surface 54. An open cell foam piece 25 enrobes a portion of the first planar nonwoven 44 and a portion of the second planar nonwoven 50. The planar nonwovens are shown as wavy due to the impact of the formation means.

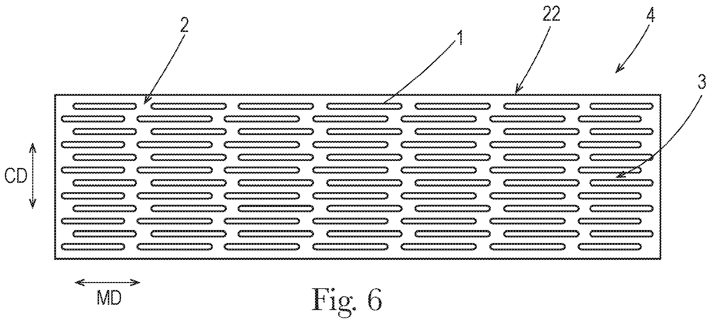

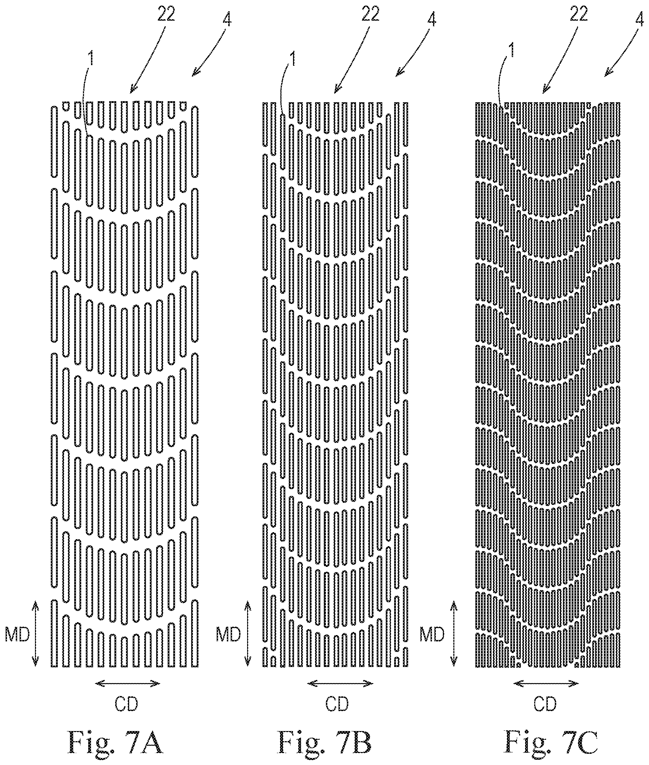

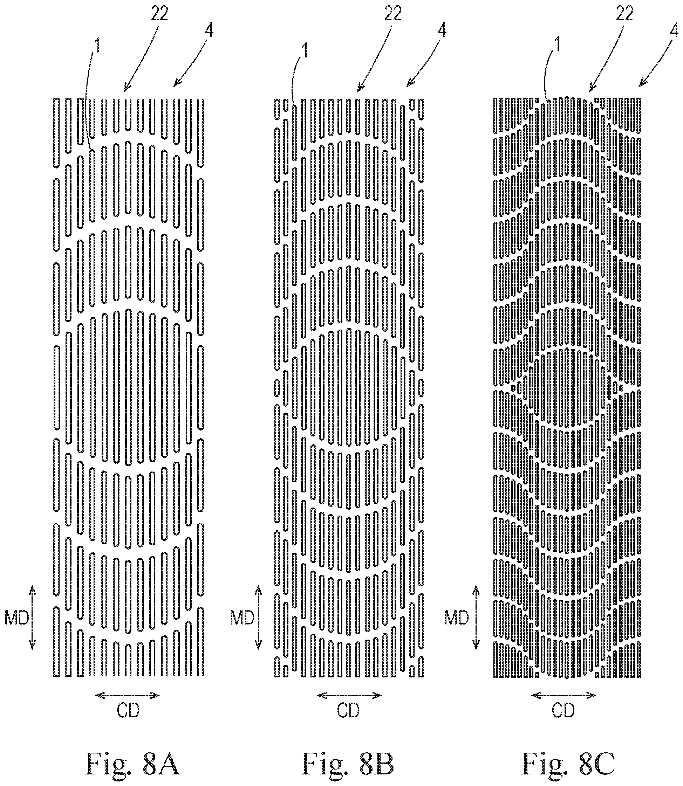

FIG. 6-8 are top views of potential patterns 4 that may be created in the heterogeneous mass 22 using formation means as described above. As shown in the FIG. 6, the canals 1 may be discontinuous such that the foam is continuous along the CD 2 and MD 3 directions. FIG. 7a-c and FIG. 8a-c represent additional optional patterns 4. As shown in FIGS. 7A-C and 8A-C, a pattern 4 may be created in the heterogeneous mass 22 using formation means such that the pattern contains canals 1 and is discontinuous in one or both of the CD 2 or the MD 3 direction such that the foam may be continuous in portions of the heterogeneous mass 22.

### Test methods

### 1- Dynamic Caliper Expansion Method

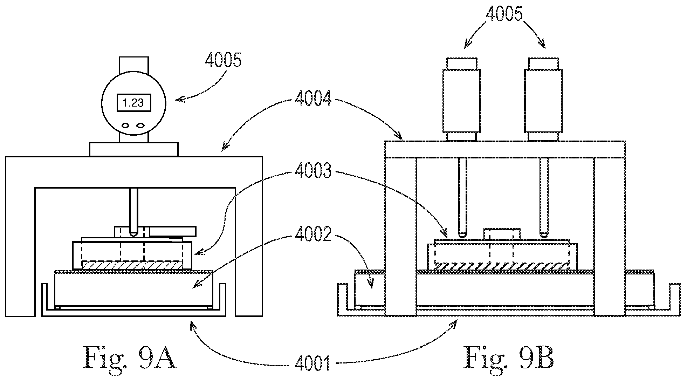

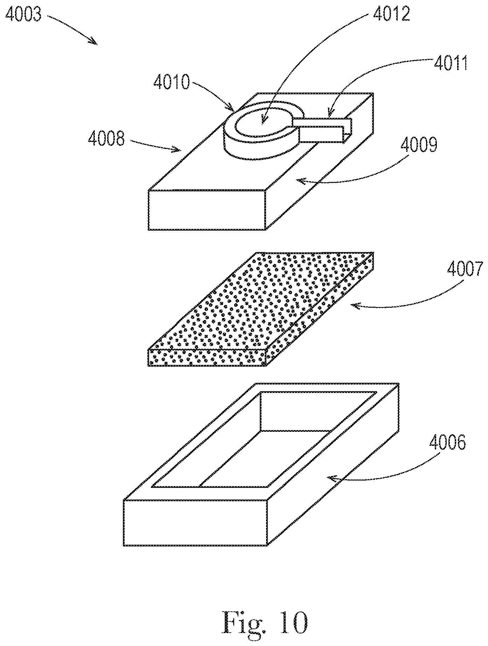

The Dynamic Caliper Expansion method measures the vertical expansion under pressure of a rectangular section of an absorbent article as fluid is introduced. Referring to Figure 9A, 9B, and 10, the test stand consist of a drainage tray 4001 to catch excess fluid, a support frame 4002 which is a base for a specimen assembly 4003, a caliper frame 4004 which supports two (2) calipers 4005, and a pump capable of delivering the test fluid at 2.40 mL/sec ± 0.01 mL/sec. The test fluid is 0.9% w/w NaCl in deionized water which is heated to 37°C ± 1C°. All test are performed in a room maintained at 23°C ± 2C° and 50% ± 2% relative humidity.

The drainage tray 4001 is made if 3.2 mm Plexiglas and is 37 cm long by 11 cm wide by 2 cm deep. The support frame 4002 is made of 9.5 mm thick Plexiglas and has outer dimensions of 35 cm long by 10 cm wide by 2 cm tall. The surface of the support frame is covered with a screen (stainless steel; 4.8 mm holes at 4 mm spacing). The caliper frame 4004 is designed to support the two calipers along the longitudinal midline of the specimen and 60 mm apart. The height of the caliper frame 4004 is tall enough that the specimen assembly can fit underneath with sufficient clearance for operation of the calipers 4005 within their dynamic range as the specimen 4007 swells. The calipers used are capable of reading to the nearest 0.001 mm with a 3 mm rounded ball as the foot (a suitable caliper is a Mitutoyo Model ID-CI50XB, or equivalent). The calipers 4005 are interfaced to a computer which records the height data at 1 Hz during the experiment.

The specimen assembly 4003 consists of a rectangular specimen frame 4006 made of 6.35 mm Plexiglas with the inside dimensions of 70.0 mm long by 50.0 mm wide by 10.0 mm deep. The specimen cover 4008 consist of a metal plate 4009 that is 69.0 mm long by 49.0 mm wide by 15.0 mm thick with a 20.0 mm diameter through-hole 4012 centered along the lateral and longitudinal axis. On top of the plate, a 20.0 mm I.D. by 30.0 mm O.D by 10.0 deep ring 4010 is adhered to the top of the plate 4009 and centered over the through-hole to give a total fluid column height of 25.0 mm. A 5.0 mm wide by 10.0 mm deep by 20.0 mm long spout 4011 permits fluid from the fluid column to overflow to outside of the specimen assembly. The overall mass of the specimen cover 4008, along with the downward force applied by the 2 calipers 4005, is such as to apply a total pressure of 0.69 kPa to the specimen 4007.

Samples are conditioned at 23°C ± 2C° and 50% ± 2% relative humidity for at least 2 hours before testing. Place the article body facing side up on a bench. On the article identify the intersection of the longitudinal midline and the lateral midline. Using a rectangular cutting die, cut a specimen 70.0 mm in the longitudinal direction by 50.0 mm in the lateral direction, centered at the intersection of the midlines.

Place the support frame 4002 within the drainage tray 4001. Place the rectangular specimen frame 4006 onto the support frame 4002 at its center. Place the specimen cover 4003 within the specimen frame without a specimen. Position the caliper frame 4004 overtop the specimen assembly 4003 with the caliper's feet resting along the longitudinal midline of the specimen cover 4003 with each foot 30.0 mm away from the center of the specimen assembly. Zero the calipers. Lift the caliper frame 4004 with calipers, from overtop the specimen assembly 4003. Gently place the specimen 4007 into the specimen frame allowing it to rest on the support frame 4002. Gently place the specimen cover 4003 on top of the specimen. The specimen cover 4003 should be able to freely move vertically within the specimen frame 4006. Position the caliper frame 4004 overtop the specimen assembly 4003 with the caliper's feet resting along the longitudinal midline of the specimen cover 4003 with each foot 30.0 mm away from the center of the specimen assembly. Read the height from both calipers and record their average as the specimen's Initial Dry Caliper to the nearest 0.0001 mm.

Zero the calipers. Program the pump to deliver 2.40 mL/sec ± 0.01 mL/sec. Start the pump and allow to run for approximately 2 min to ensure the fluid in the transfer tube is at 37°C ± 1C°. Start the height data collection at time 0. After 10 sec, begin to introduce the test fluid into the fluid column 4012 of the specimen assembly. Flow is continued up to time 5.0 min with any excess fluid being diverted away from the specimen assembly via the spout 4011.

Calculate the Dynamic Caliper Expansion for each time point taken as the average of the two caliper heights at each time point. Plot a curve of the Dynamic Caliper Expansion (mm) versus time (s) for these individual averages. Dynamic Caliper Expansion can then be read for any given time (e.g., 1.0, 2.0, 3.0, 4.0, 5.0 min etc) based on the curve and record to the nearest 0.001 mm. The % The measure is repeated for a total of five (5) replicate articles and Dynamic Caliper Expansion (mm) for any chosen time is reported as the arithmetic mean to the nearest 0.001 mm. The Initial Dry Caliper (mm) for the five (5) replicates is reported as the arithmetic mean to the nearest 0.001 mm. The caliper expansion as a percent (%) of the Specimins Initial Dry Caliper can be determined for any given time (e.g., 1.0 or 5.0 min. etc) by dividing the average Dynamic Caliper Expansion for example at time equals 1.0 minute or 5.0 minutes by the specimen's Initial Dry Caliper.

### 2 - Acquisition Speed and Rewet - SABAP test

The SABAP (Speed of Acquisition with Balloon Applied Pressure) test is designed to measure the speed at which 0.9% saline solution is absorbed into an absorbent article which is compressed at 2.07 kPa. A known volume is introduced four times, each successive dose starting five (5) minutes after the previous dose has absorbed. Times needed to absorb each dose are recorded. Subsequent to the acquisition test, PACORM (Post Acquisition Collagen Rewet Method) is performed. The test comprises measuring the mass of fluid expressed from the article under pressure after loading by the SABAP protocol. Collagen sheets are used as the rewet substrate. A suitable collagen is Naturin Coffi collagen sheets (available Naturin GmbH & KG, Germany) or equivalent. Upon receipt, the collagen sheets are stored at about 23 °C ± 2 C° and about 50% ± 2 % relative humidity. All testing is performed in a room also maintained at about 23 °C ± 2 C° and about 50% ± 2 % relative humidity.

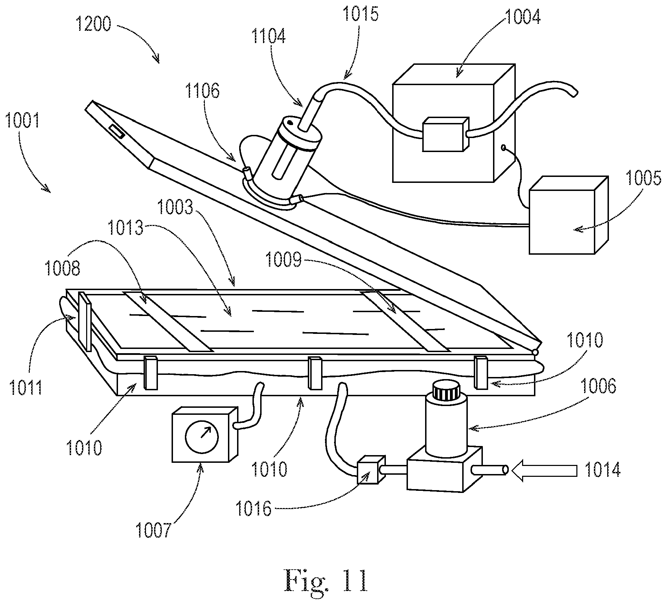

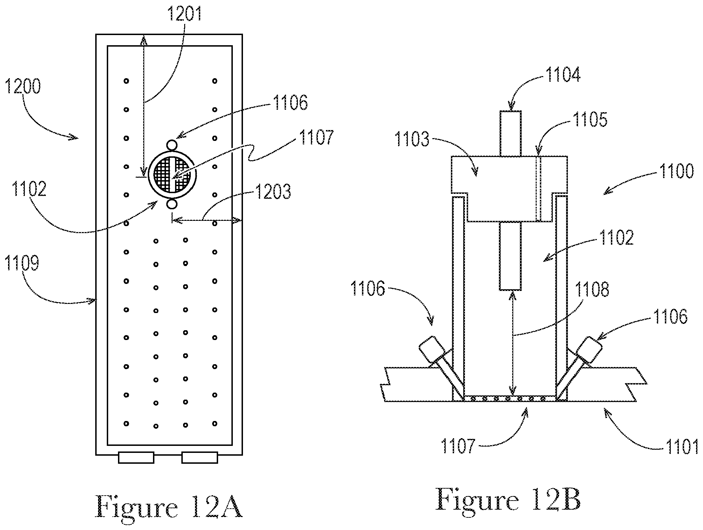

The SABAP apparatus is depicted in Figures 11 and 12A-B. It consists of a bladder assembly 1001 and a top plate assembly 1200 which includes the deposition assembly 1100. A controller 1005 is used to 1) monitor the impedance across the electrodes 1106, recording the time interval 0.9% saline solution is in the cylinder 1102, 2) interface with the liquid pump 1004 to start/stop dispensing, and 3) time intervals between dosing. The controller is capable of recording time events to ± 0.01 sec. A house air supply 1014 is connected to the pressure regulator 1006 capable of delivering air at a suitable flow/pressure to maintain 2.07 kPa in the bladder assembly. A liquid pump 1004 (Ismatec MCP-Z gear pump, available from Cole Palmer, Vernon Hills, IL or equivalent) capable of delivering a flow of 10-80 mL at a rate of 3-15 mL/s is attached to the steel tube 1104 of the deposition assembly 1100 via tygon tubing 1015.

The bladder assembly 1001 is constructed of 12.7 mm Plexiglas with an overall dimension of 80 cm long by 30 cm wide by 5 cm tall. A manometer 1007 to measure the pressure inside the assembly and a pressure gauge 1006 to regulate the introduction of air into the assembly are installed through two holes through the right side. The bladder 1013 is assembled by draping a 50 mm by 100 mm piece of silicone film, (thickness 0.02", Shore A durometer value of 20, available as Part# 86435K85 from McMaster-Carr, Cleveland, OH) over the top of the box with enough slack that the latex touches the bottom of the box at its center point. An aluminum frame 1003 with a flange is fitted over the top of the latex and secured in place using mechanical clamps 1010. When in place the assembly should be leak free at a pressure of 3.45 kPa. A front 1008 and back 1009 sample support 5 cm by 30 cm by 1 mm are used to anchor the sample. The article is attached to the top surface of the sample supports by either adhesive tape or mechanical "hook" fasteners. These supports can be adjusted along the length of the aluminum frame 1003 via a simple pin and hole system to accommodate different size absorbent articles and to correctly align their loading point.

The top plate assembly 1200 is constructed of an 80 cm by 30 cm piece of 12.7 mm Plexiglas reinforced with an aluminum frame 1109 to enhance rigidity. The deposition assembly 1100 is centered 30 cm (1201) from the front of the plate assembly and 15 cm (1203) from either side. The deposition assembly is constructed of a 50.8 mm O.D. Plexiglas cylinder 1102 with a 38.1 mm I.D. The cylinder is 100 mm tall and is inserted through the top plate 1101 and flush with the bottom of the plate 1101. Two electrodes 1106 are inserted though the top plate and cylinder and exit flush with the inner wall of the cylinder immediately above the cylinders bottom surface. A nylon screen 1107 cut into two semicircles are affixed flush with the bottom of the cylinder such that the sample cannot swell into the cylinder. The cylinder is topped with a loose-fitting nylon cap 1103. The cap has a 6.35 mm O.D. steel tube 1104 inserted through its center. When the cap is in place, the bottom of the tube ends 20 mm above (1108) the screen 1107. The cap also has an air hole 1105 to ensure negative pressure does not impede the absorption speed. In addition, the top plate has forty-four (44) 3.2 mm diameter holes drilled through it distributed as shown in Figure 12. The holes are intended to prevent air from being trapped under the top plate as the bladder is inflated but not to allow fluid to escape. The top plate assembly 1200 is connected to the bladder assembly 1001 via two hinges 1012. During use the top assembly is closed onto the bladder assembly and locked into place using a mechanical clamp 1011.

The PACORM equipment consist of a Plexiglas disk 60.0 mm in diameter and 20 mm thick and a confining weight that rest upon it. The mass of the disk and confining weight combined is 2000 g ± 2 g. Collagen is die cut into 70.0 mm circles and stacks of four (4) assembled for use during rewet testing. Measure and record the mass of the dry collagen stack and record to the nearest 0.0001 g.

Samples are conditioned at 23 °C ± 2 C° and about 50% ± 2 % relative humidity for two hours prior to testing. The article is first prepared by excising any inner or outer leg cuffs, waist caps, elastic ears or side panels, taking care not to disturb the top sheet that resides above the article's core region. Place the article flat onto a lab bench and identifying the intersection of the longitudinal and lateral centerlines of the article.

Attach the front end of the article to the top surface of the front sample plate 1008 by either adhesive tape or mechanical "hook" fasteners with the top sheet facing upward. The placement is such that just the chassis and not the absorptive core overlays the plate. The sample plate 1008 is attached to the aluminum frame 1003 such that the absorbent article will be centered longitudinally and laterally within the cylinder 1102 when the top plate assembly has been closed. The back end of the article is secured to the back sample plate 1009 by either adhesive tape or mechanical "hook" fasteners, once again ensuring that only the chassis and not the absorptive core overlays the plate. The back sample plate 1009 is then attached to the aluminum frame 1003 such that the article is taunt but not stretched. The top plate assembly is closed and fastened, and the bladder is inflated to 2.07 kPa ± 0.07 kPa.

0.9% w/v saline solution is prepared by weighing 9.0 g ± 0.05g of NaCl into a weigh boat, transferring it into a 1L volumetric flask and diluting to volume with de-ionized water. The pump 1004 is primed then calibrated to deliver 20 mL at 5 mL/sec. Volume and flow rate must be within ± 2% of target. The cap 1103 is placed into the cylinder 1102. The controller 1005 is started, which in turn delivers the first dose of 0.9% saline solution. After the volume has been absorbed, the controller waits for 5.0 minutes before addition of the next dose. This cycle is repeated for a total of four doses. If the fluid leaks out of or around the product (i.e., is not absorbed into the article) then the test is aborted. Also if any acquisition time exceeds 1200 sec, the test is aborted. Acquisition times are recorded by the controller for each dose to the nearest 0.01 sec.

After the test is complete (i.e., 5 min after the last dose is absorbed), the pressure relief valve 1016 is opened to deflate the bladder and the sample article removed from the bladder system for PACORM (Post Acquisition Collagen Rewet Method) evaluation.

Within 30 sec, place the specimen flat on a bench top, place a pre-weighed stack of collagen centered at the longitudinal and lateral midpoint of the article, place a Plexiglas disk centered onto the collagen stack, and gently place confining weight onto the disk. Wait for 15.0 sec ± 0.5 sec and remove the weight. Immediately measure the mass of the wet collagen and record to the nearest 0.0001 g. Calculate the rewet value as the difference between the wet and dry weight of the stack and record to the nearest 0.0001g.

In like fashion run a total of five (5) replicates for each article to be evaluated. Calculate and report the acquisition rates mL/sec for each dose as the geometric mean to the nearest 0.01 mL/sec. Using the caliper from the Dry Caliper method described herein calculate the Acquisition Rate (mL/sec) divided by the Initial Caliper (mm) and report to the nearest 0.1 mL/sec/mm. Calculate the Rewet for the five replicates as the geometric mean to the nearest 0.0001 g.

### 3- Bunch Compression Test

Bunched Compression of a sample is measured on a constant rate of extension tensile tester (a suitable instrument is the MTS Alliance using Testworks 4.0 software, as available from MTS Systems Corp., Eden Prairie, MN, or equivalent) using a load cell for which the forces measured are within 10% to 90% of the limit of the cell. All testing is performed in a room controlled at 23°C +/- 3°C and 50% +/- 2% relative humidity. The test can be performed wet or dry.

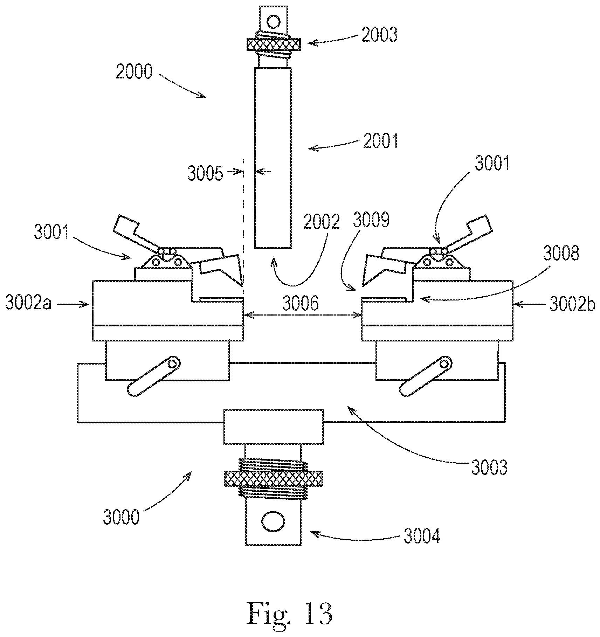

Referring to fig. 13, the bottom stationary fixture 3000 consists of two matching sample clamps 3001 each 100 mm wide each mounted on its own movable platform 3002a, 3002b. The clamp has a "knife edge" 3009 that is 100 mm long, which clamps against a 1 mm thick hard rubber face 3008. When closed, the clamps are flush with the interior side of its respective platform. The clamps are aligned such that they hold an un-bunched specimen horizontal and orthogonal to the pull axis of the tensile tester. The platforms are mounted on a rail 3003 which allows them to be moved horizontally left to right and locked into position. The rail has an adapter 3004 compatible with the mount of the tensile tester capable of securing the platform horizontally and orthogonal to the pull axis of the tensile tester. The upper fixture 2000 is a cylindrical plunger 2001 having an overall length of 70 mm with a diameter of 25.0 mm. The contact surface 2002 is flat with no curvature. The plunger 2001 has an adapter 2003 compatible with the mount on the load cell capable of securing the plunger orthogonal to the pull axis of the tensile tester.

Samples are conditioned at 23°C +/- 3°C and 50% +/- 2% relative humidity for at least 2 hours before testing. When testing a whole article, remove the release paper from any panty fastening adhesive on the garment facing side of the article. Lightly apply talc powder to the adhesive to mitigate any tackiness. If there are cuffs, excise them with scissors, taking care not to disturb the top sheet of the product. Place the article, body facing surface up, on a bench. On the article identify the intersection of the longitudinal midline and the lateral midline. Using a rectangular cutting die, cut a specimen 100 mm in the longitudinal direction by 80 mm in the lateral direction, centered at the intersection of the midlines. When testing just the absorbent body of an article, place the absorbent body on a bench and orient as it will be integrated into an article, i.e., identify the body facing surface and the lateral and longitudinal axis. Using a rectangular cutting die, cut a specimen 100 mm in the longitudinal direction by 80 mm in the lateral direction, centered at the intersection of the midlines.

The specimen can be analyzed both wet and dry. The dry specimen requires no further preparation. The wet specimens are dosed with one of two test solutions: 10.00 mL ± 0.01 mL of a 0.9% w/v saline solution (i.e., 9.0 g of NaCl diluted to 1L deionized water) or 7.00 mL ± 0.01 mL 10% w/v saline solution (100.0 g of NaCl diluted to 1L deionized water). The dose is added using a calibrated Eppendorf-type pipettor, spreading the fluid over the complete body facing surface of the specimen within a period of approximately 3 sec. The wet specimen is tested 10.0 min ± 0.1 min after the dose is applied.

Program the tensile tester to zero the load cell, then lower the upper fixture at 2.00 mm/sec until the contact surface of the plunger touches the specimen and 0.02 N is read at the load cell. Zero the crosshead. Program the system to lower the crosshead 15.00 mm at 2.00 mm/sec then immediately raise the crosshead 15.00 mm at 2.00 mm/sec. This cycle is repeated for a total of five cycles, with no delay between cycles. Data is collected at 100 Hz during all compression/decompression cycles.

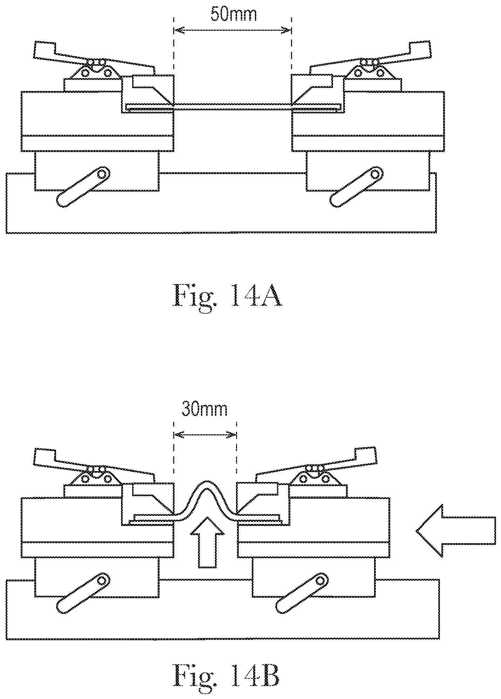

Position the left platform 3002a 2.5 mm from the side of the upper plunger (distance 3005). Lock the left platform into place. This platform 3002a will remain stationary throughout the experiment. Align the right platform 3002b 50.0 mm from the stationary clamp (distance 3006). Raise the upper probe 2001 such that it will not interfere with loading the specimen. Open both clamps. Referring to Figure 14A, place the specimen with its longitudinal edges (i.e., the 100 mm long edges) within the clamps. With the specimen laterally centered, securely fasten both edges. Referring to Figure 14B, move the right platform 3002b toward the stationary platform 3002a a distance 20.0 mm. Allow the specimen to bow upward as the movable platform is positioned. Manually lower the probe 2001 until the bottom surface is approximately 1 cm above the top of the bowed specimen.

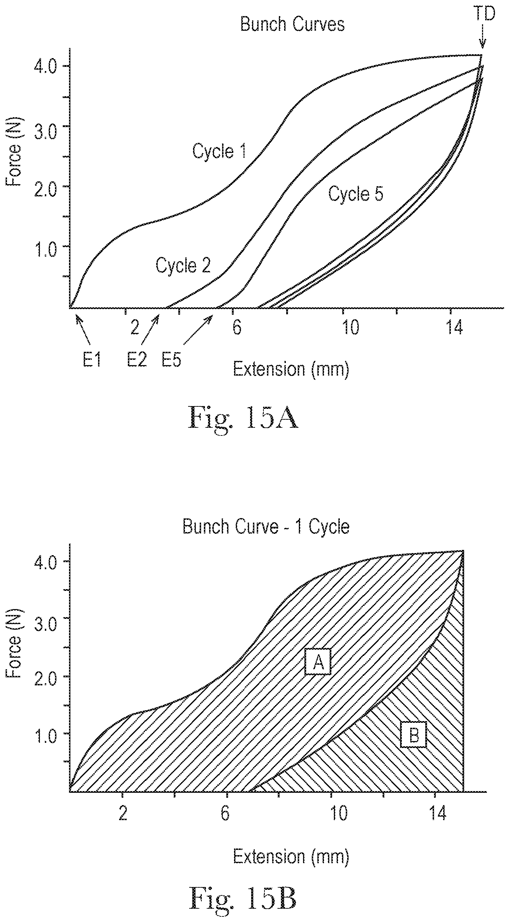

Start the test and collect displacement (mm) verses force (N) data for all five cycles. Construct a graph of Force (N) versus displacement (mm) separately for all cycles. A representative curve is shown in Figure 15A. From the curve record the Maximum Compression Force for each Cycle to the nearest 0.01N. This is the "dry peak stiffness". Calculate the % Recovery between the First and Second cycle as (TD-E2)/(TD-E1)*100 where TD is the total displacement and E2 is the extension on the second compression curve that exceeds 0.02 N. Record to the nearest 0.01%. In like fashion calculate the % Recovery between the First Cycle and other cycles as (TD-Ei)/(TD-E1)*100 and report to the nearest 0.01%. Referring to Figure 15B, calculate the Energy of Compression for Cycle 1 as the area under the compression curve (i.e., area A+B) and record to the nearest 0.1 mJ. Calculate the Energy Loss from Cycle 1 as the area between the compression and decompression curves (i.e., Area A) and report to the nearest 0.1 mJ. Calculate the Energy of Recovery for Cycle 1 as the area under the decompression curve (i.e. Area B) and report to the nearest 0.1 mJ. In like fashion calculate the Energy of Compression (mJ), Energy Loss (mJ) and Energy of Recovery (mJ) for each of the other cycles and record to the nearest 0.1 mJ

For each sample, analyze a total of five (5) replicates and report the arithmetic mean for each parameter. All results are reported specifically as dry or wet including test fluid (0.9% or 10%).

### 4- Dry Caliper

Dry Caliper (thickness) of a specimen or product is measured using a calibrated digital linear caliper (e.g., Ono Sokki GS-503 or equivalent) fitted with a 25.4 mm diameter foot with an anvil that is large enough that the specimen can lie flat. The foot applies a confining pressure of 2.07 kPa to the specimen. Zero the caliper foot against the anvil. Lift the foot and insert the specimen flat against the anvil and lower the foot at about 5 mm/sec onto the specimen. Read the caliper (mm) 5.0 sec after resting the foot on the sample and record to the nearest 0.01 mm.

## Claims

1. An absorbent article, comprising:
a. a fluid permeable topsheet;
b. a backsheet; and
c. an absorbent element disposed between the topsheet and the backsheet, wherein the absorbent element comprises two or more layers wherein an upper layer is positioned closer to the topsheet and a lower layer is positioned closer to the backsheet, and
wherein the upper layer is a heterogeneous mass layer comprising a longitudinal axis, a lateral axis, a vertical axis, one or more enrobeable elements, and one or more discrete open-cell foam pieces, wherein said enrobeable elements are fibers, preferably synthetic fibers, and wherein one or more of said discrete open-cell foam pieces enrobe said enrobeable elements,
wherein the lower layer comprises of a substrate comprising superabsorbent polymer particles, and the superabsorbent polymer particles are disposed on the substrate layer, preferably a nonwoven substrate layer;
d. wherein the absorbent article has a caliper expansion measured at five minutes according to the dynamic caliper expansion test described herein;
e. wherein the absorbent article has a dry peak stiffness measured according to the bunch compression test described herein; and
f. wherein a ratio of the caliper expansion to the dry peak stiffness is at least 0.5%/N.

2. The absorbent article according to claim 1, wherein the ratio of the caliper expansion to the dry peak stiffness is at least 0.75%/N.

3. The absorbent article according to claims 1 or 2, wherein the ratio of the caliper expansion to the dry peak stiffness is from 0.5%/N to 5%/N.

4. The absorbent article according to any of the preceding claims, wherein absorbent article has a caliper expansion, measured at five minutes according to the dynamic caliper expansion test described herein, that is at least 125%; and wherein the absorbent article has a dry peak stiffness, measured according to the bunch compression test described herein, of 10 N or less.

5. The absorbent article according to any of the preceding claims, wherein absorbent article has a caliper expansion, measured at five minutes according to the dynamic caliper expansion test described herein, that is at least 75%; and wherein the absorbent article has a dry peak stiffness, measured according to the bunch compression test described herein, of 7 N or less.

6. The absorbent article according to any of the preceding claims, wherein said open cell foam pieces are in the form of stripes parallel to a longitudinal axis, a lateral axis, a diagonal axis, or a combination thereof.

7. The absorbent article according to any of the preceding claims, wherein the open-cell foam pieces comprise HIPE foam.

8. The absorbent article according to any of the preceding claims, wherein the absorbent article has a rewet value, as measure according to the SABAP test described herein, of 0.1 g or less.

9. The absorbent article according to any of the preceding claims, wherein the absorbent article has an acquisition rate, as measured according to the SABAP test described herein, of from 1 ml/s to 6 ml/s.

10. The absorbent article according to any of the preceding claims, wherein the absorbent article comprises a caliper expansion, measured at 1 minute according to the dynamic caliper expansion test described herein, of from 150% to 250%.

## Patentansprüche

1. Absorptionsartikel, umfassend:
a. eine fluiddurchlässige Oberschicht;
b. eine Unterschicht; und
c. ein Absorptionselement, das zwischen der Oberschicht und der Unterschicht angebracht ist, wobei das Absorptionselement zwei oder mehr Schichten umfasst, wobei eine obere Schicht näher an der Oberschicht angeordnet ist und eine untere Schicht näher an der Unterschicht angeordnet ist, und
wobei die obere Schicht eine heterogene Massenschicht ist, die eine Längsachse, eine Querachse, eine vertikale Achse, ein oder mehrere umhüllbare Elemente und ein oder mehrere diskrete offenzellige Schaumstoffstücke umfasst, wobei die umhüllbaren Elemente Fasern, vorzugsweise synthetische Fasern, sind und wobei eines oder mehrere der diskreten offenzelligen Schaumstoffstücke die umhüllbaren Elemente umhüllen,
wobei die untere Schicht ein Substrat umfasst, das Superabsorberpolymerteilchen umfasst, und die Superabsorberpolymerteilchen auf der Substratschicht, vorzugsweise einer Vliessubstratschicht, angebracht sind;
d. wobei der Absorptionsartikel eine Dickenausdehnung aufweist, die gemäß der hierin beschriebenen dynamischen Dickenausdehnungsprüfung bei fünf Minuten gemessen wird;
e. wobei der Absorptionsartikel eine Trockenspitzensteifigkeit aufweist, die gemäß der hierin beschriebenen Bündelkompressionsprüfung gemessen wird; und
f. wobei ein Verhältnis der Dickenausdehnung zu der Trockenspitzensteifigkeit mindestens 0,5 %/N beträgt.

2. Absorptionsartikel nach Anspruch 1, wobei das Verhältnis der Dickenausdehnung zu der Trockenspitzensteifigkeit mindestens 0,75 %/N beträgt.

3. Absorptionsartikel nach Anspruch 1 oder 2, wobei das Verhältnis der Dickenausdehnung zu der Trockenspitzensteifigkeit von 0,5 %/N bis 5 %/N beträgt.

4. Absorptionsartikel nach einem der vorstehenden Ansprüche, wobei der Absorptionsartikel eine Dickenausdehnung aufweist, gemessen bei fünf Minuten gemäß der hierin beschriebenen dynamischen Dickenausdehnungsprüfung, die mindestens 125 % beträgt; und wobei der Absorptionsartikel eine Trockenspitzensteifigkeit, gemessen gemäß der hierin beschriebenen Bündelkompressionsprüfung, von 10 N oder weniger aufweist.

5. Absorptionsartikel nach einem der vorstehenden Ansprüche, wobei der Absorptionsartikel eine Dickenausdehnung aufweist, gemessen bei fünf Minuten gemäß der hierin beschriebenen dynamischen Dickenausdehnungsprüfung, die mindestens 75 % beträgt; und wobei der Absorptionsartikel eine Trockenspitzensteifigkeit, gemessen gemäß der hierin beschriebenen Bündelkompressionsprüfung, von 7 N oder weniger aufweist.

6. Absorptionsartikel nach einem der vorstehenden Ansprüche, wobei die offenzelligen Schaumstoffstücke in Form von Streifen parallel zu einer Längsachse, einer Querachse, einer Diagonalachse oder einer Kombination davon vorliegen.

7. Absorptionsartikel nach einem der vorstehenden Ansprüche, wobei die offenzelligen Schaumstoffstücke HIPE-Schaumstoff umfassen.

8. Absorptionsartikel nach einem der vorstehenden Ansprüche, wobei der Absorptionsartikel einen Rücknässungswert, wie gemäß der hierin beschriebenen SABAP-Prüfung gemessen, von 0,1 g oder weniger aufweist.

9. Absorptionsartikel nach einem der vorstehenden Ansprüche, wobei der Absorptionsartikel eine Aufnahmerate, wie gemäß der hierin beschriebenen SABAP-Prüfung gemessen, von 1 ml/s bis 6 ml/s aufweist.

10. Absorptionsartikel nach einem der vorstehenden Ansprüche, wobei der Absorptionsartikel eine Dickenausdehnung, gemessen bei 1 Minute gemäß der hierin beschriebenen dynamischen Dickenausdehnungsprüfung, von 150 % bis 250 % umfasst.

## Revendications

1. Article absorbant, comprenant :
a. une feuille de dessus perméable aux fluides ;
b. une feuille de fond ; et
c. un élément absorbant disposé entre la feuille de dessus et la feuille de fond, dans lequel l'élément absorbant comprend deux couches ou plus dans lequel une couche supérieure est positionnée plus près de la feuille de dessus et une couche inférieure est positionnée plus près de la feuille de fond, et
dans lequel la couche supérieure est une couche de masse hétérogène comprenant un axe longitudinal, un axe latéral, un axe vertical, un ou plusieurs éléments enrobables, et une ou plusieurs pièces individuelles de mousse à alvéoles ouvertes, dans lequel lesdits éléments enrobables sont des fibres, de préférence des fibres synthétiques, et dans lequel une ou plusieurs desdites pièces individuelles de mousse à alvéoles ouvertes enrobent lesdits éléments enrobables,
dans lequel la couche inférieure comprend un substrat comprenant des particules de polymère superabsorbant, et les particules de polymère superabsorbant sont disposées sur la couche de substrat, de préférence une couche de substrat non tissé ;
d. dans lequel l'article absorbant a une expansion de calibre mesurée à cinq minutes selon le test d'expansion dynamique de calibre décrit ici ;
e. dans lequel l'article absorbant a un pic de rigidité à sec mesuré selon le test de compression de paquets décrit ici ; et
f. dans lequel un rapport de l'expansion de calibre au pic de rigidité à sec vaut au moins 0,5 %/N.

2. Article absorbant selon la revendication 1, dans lequel le rapport de l'expansion de calibre au pic de rigidité à sec vaut au moins 0,75 %/N.

3. Article absorbant selon les revendications 1 ou 2, dans lequel le rapport de l'expansion de calibre au pic de rigidité à sec va de 0,5 %/N à 5 %/N.

4. Article absorbant selon l'une quelconque des revendications précédentes, dans lequel l'article absorbant a une expansion de calibre, mesurée à cinq minutes selon le test d'expansion dynamique de calibre décrit ici, qui vaut au moins 125 % ; et dans lequel l'article absorbant a un pic de rigidité à sec, mesuré selon le test de compression de paquets décrit ici, de 10 N ou moins.

5. Article absorbant selon l'une quelconque des revendications précédentes, dans lequel l'article absorbant a une expansion de calibre, mesurée à cinq minutes selon le test d'expansion dynamique de calibre décrit ici, qui vaut au moins 75 % ; et dans lequel l'article absorbant a un pic de rigidité à sec, mesuré selon le test de compression de paquets décrit ici, de 7 N ou moins.

6. Article absorbant selon l'une quelconque des revendications précédentes, dans lequel lesdites pièces de mousse à alvéoles ouvertes sont sous la forme de bandes parallèles à un axe longitudinal, un axe latéral, un axe diagonal, ou une combinaison de ceux-ci.

7. Article absorbant selon l'une quelconque des revendications précédentes, dans lequel les pièces de mousse à alvéoles ouvertes comprennent de la mousse HIPE.

8. Article absorbant selon l'une quelconque des revendications précédentes, dans lequel l'article absorbant a une valeur de remouillage, telle que mesurée selon le test SABAP décrit ici, de 0,1 g ou moins.

9. Article absorbant selon l'une quelconque des revendications précédentes, dans lequel l'article absorbant a une vitesse de recueil, telle que mesurée selon le test SABAP décrit ici, allant de 1 ml/s à 6 ml/s.

10. Article absorbant selon l'une quelconque des revendications précédentes, dans lequel l'article absorbant comprend une expansion de calibre, mesurée à 1 minute selon le test d'expansion dynamique de calibre décrit ici, allant de 150 % à 250 %.
